# EUROPÄISCHE PATENTSCHRIFT

(11) **EP 0 772 617 B1**
(45) Veröffentlichungstag und Bekanntmachung des Hinweises auf die Patenterteilung: **23.06.1999**
(21) Anmeldenummer: 95926894.7
(22) Anmeldetag: 14.07.1995
(51) Int. Cl.: C07H 7/02, C07H 3/04, A61K 31/70

(54) **7-O-CARBAMOYL-HEPTOSE-DERIVATE, VERFAHREN ZU DEREN HERSTELLUNG, UND DEREN VERWENDUNG SOWIE SCREENING-VERFAHREN ZU DEREN BESTIMMUNG**
7-O-CARBAMOYL HEPTOSE DERIVATIVES, PROCESS FOR THEIR PRODUCTION AND THEIR USE AND SCREENING PROCESS FOR THEIR DETECTION
DERIVES DE 7-O-CARBAMYLE-HEPTOSE, LEUR PROCEDE DE PREPARATION, LEUR UTILISATION ET PROCEDE DE DEPISTAGE PERMETTANT DE LES DETECTER

(30) Priorität: 15.07.1994 DE 4425098
(43) Veröffentlichungstag der Anmeldung: 14.05.1997
(73) Patentinhaber: Forschungsinstitut Borstel Institut für Experimentelle Biologie und Medizin, D-23845 Borstel (DE)
(72) Erfinder: ZÄHRINGER, Ulrich, D-23845 Borstel (DE); BECKMANN, Frank, D-23845 Borstel (DE); MOLL, Hermann, D-23845 Borstel (DE)
(74) Vertreter: Merkle, Gebhard
(86) Internationale Anmeldenummer: EP9502780
(87) Internationale Veröffentlichungsnummer: WO9602550

(56) Entgegenhaltungen:
- EP-A- 0 253 912

## Beschreibung

### Gebiet der Erfindung

Die vorliegende Erfindung betrifft 7-O-Carbamoyl-Heptose-Derivate, ein Verfahren zu deren Herstellung und deren Verwendung zur Herstellung von Reagenzien und Zusammensetzungen für die Diagnose und Therapie von Pseudomonasinfektionen bei Mensch und Tier sowie ein Screening-Verfahren zu deren Bestimmung in Gram-negativen Bakterien.

### Hintergrund der Erfindung

Bakterien der Familie Pseudomonadaceae sind ubiquitär vorkommende Gram-negative Keime, deren Pathogenität für den Menschen normalerweise schwach entwickelt ist. P. *aeruginosa* dagegen ist eine humanpathogene Art und tritt häufig bei Wundinfektionen und dort besonders als Sekundärinfektion bei Verbrennungen höheren Grades der Haut und bei Mittelohrvereiterungen auf. Bei immungeschwächten Patienten und bei der zystischen Fibrose sind besonders die antibiotika-resistenten Pseudomonaden von herausragender medizinischer Bedeutung [McManus et al.. *J*. *Trauma 21* (1981) 753-763, Bodey et al.. *Rev. inf. Dis.. 5*(1983). 279-313, Winkler et al., *Klin Wochenschrift, 63* (1985) 490-498].

Taxonomisch stellen Pseudomonaden eine sehr heterogene Familie dar. Ihre speziesbedingte Heterogenität stellt eine wesentliche Erschwernis bei der medizinischen Diagnose und der Therapie der Pseudomonasinfektionen dar. Aus diesem Grunde wurde erst vor kurzer Zeit vorgeschlagen, die-Familie der Pseudomonadaceae in fünf verschiedene Untergruppen zu unterteilen [N.J. Palleroni, *Antonie Van Leeuwenhoek*, 64 (1993) 231-251]. Zur ersten Gruppe (RNA-Gruppe 1) gehören u.a. *P*. *aeruginosa. P.fluorescens* und *P*. *putida.* Die zweite Gruppe (RNA-Gruppe 2) umfaßt die pflanzen- und tierpathogenen Pseudomonaden (z. B. P. *plantarii*) und wird jetzt Burkholderia bezeichnet. Schließlich unterscheidet man noch Comamonas (Gruppe 3) und die Purpurbakterien (Gruppe 4) die weiterhin als "Pseudomonas" bezeichnet werden (z. B. *P*, *diminuta. P. vesicularis*) und letztlich noch Xanthomonas (RNA-Gruppe 5).

Pseudomonaden sind darüber hinaus auch noch aus anderen Gründen von biomedizinischem Interesse. Sie sind äußerst resistent gegenüber Antibiotika [A.M. Kropinski et al.. *Antimicrob. Agents Chemother., 36*(1985) 58-73]. Es existieren Hinweise, daß diese Antibiotikaresistenz mit der Struktur der Zellwandmembran, d. h. mit einer hohen Dichte an negativ geladenen Phosphatgruppen von Molekülen der äußeren Zellwandmembran einhergeht [R.E.W, Hancock et al., *in*: *Bacterial Cell Wall,* J.M. Ghuysen und R. Hakenbeck(eds.), ElsevierAmsterdam, 1994. 263-279). Solche Oberflächenstrukturen sind bei allen Gram-negativen Bakterien im wesentlichen integrale Proteine der Zellmembran (OMP, Porine), und das Lipopolysaccharid (LPS, Endotoxin). Diese Moleküle repräsentieren Antigene, welche genus-, spezies- und subspeziesspezifisch hoch immunogen sind und im Verlaufe einer Infektion serotypspezifische Antikörper induzieren, die diagnostisch wie therapeutisch von großer Bedeutung sein können.

Im Verlauf des letzten Jahrzehnts konnte ein beträchtliches Wissen über die serologischen und biologischen Eigenschaften dieser Antigene erarbeitet werden. Auch die LPS-Strukturen bzw. und dort besonders deren immunogene äußeren Komponenten (O-Ketten) von den heute bekannten 17 Serotypen sind intensiv bearbeitet und aufgeklärt worden. Alle *P. aeruginosa* O-Ketten wurden chemisch vollständig analysiert. z. T. auch synthetisiert und serologisch in verschiedene Immuno- (Fischer) und Serotypen (Lanyi, Habs) klassifiziert (N.K. Kochetkov und Yu. A. Knirel, *Sov. Sci. Rev. B. Chem., 13* (1989) 1-101]. Auf der Basis dieser Strukturkenntnis wurden verschiedene *P. aeruginosa* "Serotyping Kits" mit monoklonalen Antikörpern hergestellt. die auch heute im Handel sind. Der Nachteil dieser Antikörper-Test-Cocktails besteht darin, daß sie lediglich die bekannten Antikörper umfassen, jedoch alle monoklonalen O-spezifischen Antikörper notwendig sind um alle O-Typen zu umfassen.

Neben den seit langem bekannten Antigen-Testverfahren der immunologisch hoch spezifischen O-Ketten sind in jüngster Zeit kreuzreagierende monoklonale Antikörper entwickelt worden, deren Epitop nicht in der hochvariablen O-Kette des Llpopolysaccharides lokalisiert ist. sondern in dem weniger variablen Kern-Oligosaccharid. Da diese Strukturen wesentlich zur Funktion der äußeren Zellmembran mit beitragen, betrachtet man Kern-Oligosaccharide eher als konservative Strukturelemente des immunogenen LPS, gegen die breit kreuzschützende Antikörper hergestellt werden könnten. Dies konnte kürzlich erstmals experimentell bewiesen werden. Ein monoklonaler Antikörper (MAb) aus der Maus, dessen Spezifität gegen die Kernregion gerichtet war, zeigte breite Kreuzreaktivität und breiten Kreuzschutz gegen alle fünf *Escherichia coli* (R1, R2, R3, R4, K-12) wie auch gegen die *Salmonella minnesota* Kern-Oligosaccharidstruktur, unabhängig von der Struktur der O-Kette des jeweiligen LPS [Di Padova et al., *Infect. Immun. 61*, (1993) 3863-3872]. Dieser MAb (WN1 222-5) der IgG2a Klasse vermittelt einen breiten Kreuzschutz gegen *S. minnesota* und *E. coli* Endotoxin, jedoch nicht gegenüber *P*. *aeruginosa* oder *Klebsiella pneumoniae.* Der Grund hierfür wird der unterschiedlichen Kern-Oligosaccharidstruktur zugeschrieben. Die Kern-Oligosaccharid-Strukturen von *P*. *aeruginosa* und *K. pneumoniae* sind bisher nur unvollständig analysiert bzw. weitgehend unbekannt.

In einer Studie, bei der verschiedene Rauhform-Mutanten von *P. aeruginosa* hergestellt und deren Kern-Oligosaccharid chemisch untersucht worden waren [P.S.N. Rowe & P.M. Meadow. *Eur. J. Biochem. 132* (1983) 329-337], wurden erste Strukturvorschläge der Kernregion unterbreitet. Eine strukturelle Besonderheit war das Vorhandensein der Aminosäure Alanin (Ala) im äußeren Kern-Oligosacharid bei allen untersuchten *P. aeruginosa* Rauhform-Mutanten. Dieser Strukturvorschlag wurde erst zehn Jahre später an einer *P. aeruginosa* Mutanten [R5 (Habs 06)] mittels ¹H-NMR Spektroskopie revidiert und verbessert. [E. Altman et al., Int. Carb. Conference, Paris 1992, E. Altman et al.. 2nd IES Conference, Vienna 1992].

Jüngste Untersuchungen in unserem Labor ergaben jedoch, daß auch diese Struktur des Kern-Oligosaccharids der aus der Rauhform-Mutanten PAC1R abgeleiteten Tiefrauh-Mutanten [R5 (Habs 06)] von *P. aeruginosa* immer noch unvollständig ist. Zunächst war bekannt, daß diese Kernregion sehr stark phosphoryliert ist. Zum anderen waren im wesentlichen die Abbaumethoden und Analysenverfahren, welche E. Altman et al.. verwendet haben, nicht geeignet, um die gemäß der vorliegenden Erfindung erstmals eingehend beschriebene 7-O-Carbamoyl-L-glycero-D-manno-Heptopyranose zu identifizieren und spektrometrisch (¹H-NMR) vermessen zu können.

### Zusammenfassung der Erfindung

Die Erfinder haben die chemische Struktur des Lipopolysaccharids von Pseudomonaden untersucht, mit dem Ziel, spezifische Mono- und Disaccharide zur Verfügung zu stellen, welche als serologische Marker bei der Diagnose und Therapie von Pseudomonasinfektionen eingesetzt werden können.

Bei diesen Untersuchungen konnte die chemische Struktur einer bisher unbekannten 7-O-Carbamoyl-L-glycero-D-manno-Heptopyranose im Kern-Oligosaccharid des LPS von Pseudomonaden der RNA-Gruppe 1 analysiert und vollständig charakterisiert werden. In anderen Gram-negativen Bakterien außer Pseudomonaden der RNA-Gruppe 1 (z. B. in allen Enterobacteriaceae) konnte diese Heptopyranose nicht gefunden werden.

Gegenstand der Erfindung sind daher 7-O-Carbamoyl-Heptose-Derivate der allgemeinen Formel (I) 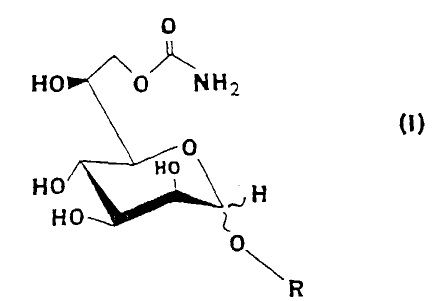 worin R den Substituenten R¹ oder eine Gruppe der allgemeinen Formel (II) bedeutet, 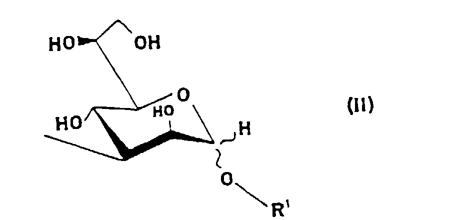 worin R¹ ein Wasserstoffatom, eine Methylgruppe oder ein für eine kovalente Kopplung geeigneter Linker-Substituent ist.

Gegenstand der Erfindung ist ferner ein Verfahren zur Herstellung der 7-O-Carbamoyl-Heptose-Derivate, welches dadurch gekennzeichnet ist, daß man ein Lipopolysaccharid von Pseudomonaden der RNA-Gruppe 1 mit Fluorwasserstoffsäure behandelt, das erhaltene Produkt bis zur Erreichung eines neutralen pH-Werts gegen Wasser dialysiert, das so dephosphorylierte Lipopolysaccharid methanolisiert. daran anschließend eine Permethylierung oder Perazetylierung durchführt, die erhaltenen 7-O-Carbamoyl-Heptose-Derivate durch Flüssigkeitschromatographie (unter Überprüfung der Reinheit durch Gas-Flüssigkeitschromatographie oder kombinierte Gas-Flüssigkeitschromatographie/Massenspektrometrie) auftrennt, und falls erwünscht, den Substituenten R¹ nach einem an sich bekannten Verfahren in die erhaltenen 7-O-Carbamoyl-Heptose-Derivate einführt.

Gegenstand der Erfindung ist weiterhin ein Screening-Verfahren zur Bestimmung der 7-O-Carbamoyl-Heptose-Derivate in Gram-negativen Bakterien, welches dadurch gekennzeichnet ist, daß man intakte Bakterien ohne vorherige Abtrennung des Lipopolysaccharids mit Fluorwasserstoffsäure behandelt, danach zur Freisetzung der Heptose-Derivate hydrolysiert bzw. methanolisiert, daran anschließend eine Permethylierung durchführt und das permethylierte Produkt mittels Gas-Flüssigkeitschromatographie bzw. kombinierter Gas-Flüssigkeitschromatographie/Massenspektrometrie analysiert.

Die 7-O-Carbamoyl-Heptose-Derivate der allgemeinen Formel (I) können zur Herstellung von Reagenzien und Zusammensetzungen für die Diagnose und Therapie von Pseudomonasinfektionen bei Mensch und Tier verwendet werden.

### Detaillierte Beschreibung der bevorzugten Ausführungsformen

Der durch R¹ in der allgemeinen Formel (II) angegebene Linker-Substituent ist vorzugsweise ein Cysteaminrest, eine Allylgruppe oder eine geradkettige oder eine verzweigtkettige C₁₋₁₈-Alkylgruppe, die eine endständige Hydroxyl-, Amino-, Carbonyl-. Carboxyl- oder Allylgruppe enthalten kann. Vorzugsweise ist R¹ ein Wasserstoffatom oder eine Methylgruppe.

Gemäß der vorliegenden Erfindung ist es gelungen, die Heptoseregion im Kern-Oligosaccharid des LPS von biomedizinisch bedeutenden Pseudomonaden analytisch zugängig zu machen und ihre phosphatfreie Struktur vollständig zu charakterisieren. Darüber hinaus ist es gemäß der Erfindung gelungen, diesen bislang unbekannten Zucker aus diesen Bakterien zu isolieren und mittels kombinierter Gas-Chromatographie/Massenspektrometrie (GC-MS) analysieren und quantifizieren zu können.

Die neue 7-O-Carbamoyl-Heptopyranose konnte bislang nur in Pseudomonaden der RNA-Gruppe 1 (insbesondere im humanpathogenen P. aeruginosa und in P. fluorescens) identifiziert werden.

Darüber hinaus kommt der neue Zucker im LPS von allen bisher untersuchten Immunotypen von *P. aeruginosa* (Fischer 2,7), unabhängig von der Struktur der O-Kette vor. Ausnahmen von dieser Regel sind bisher nicht bekannt. In den pflanzenpathogenen Pseudomonaden (z. B. *P. plantarii*)*,* die heute nicht mehr zur Gruppe der Pseudomonaden, sondern den Burkholderia zugeordnet werden, kommt die 7-O-Carbamoyl-Heptopyranose nicht im LPS vor.

Aus diesen Befunden kann eine diagnostische wie taxonomische Bedeutung der 7-O-Carbamoyl-Heptopyranose abgeleitet werden. Die 7-O-Carbamoyl-Heptopyranose fehlt ebenso in allen anderen bisher untersuchten Gram-negativen Bakterien: *Klebsiella pneumoniae,* K25; *Yersinia enterocolitica,* Mutante 490 M;, *Campylobacter jejuni* RN16 O:58. CCUG 10936; *Proteus mirabilis,* Mutante R₄₅; *Haemophilus influenzae,* B. Stamm Eagan, *Vibrio parahaemolyticus,* Serotyp O12, *Salmonella minnesota,* SF 1111, sowie *E. coli* O111 waren im Screeningverfahren bezüglich der 7-O-Carbamoyl-Heptose negativ, was die diagnostische Bedeutung dieses neuentdeckten Zuckers noch einmal unterstreicht.

Mittels dem erfindungsgemäßen Zucker ist es nun möglich geworden, die spezies-spezifische Diagnostik von Pseudomonaden und ihre taxonomische Klassifikation weiter zu verbessern.

Seitdem bekannt ist, daß monoklonale Antikörper, die gegen Epitope der inneren und äußeren Kernregion gerichtet sind, auch Wildtyp-LPS mit analogem Kernoligosaccharid erkennen können [Di Padova, F. et al.. *Infect. Immun., 61* (1993) 3863-3872 und Rietschel, e.Th. et al.. FASEB. J., 8 (1994) 217-225] ist es möglich, auf der Basis der vorliegenden Strukturaufklärung, spezies-spezifische Epitope mittels monoklonalen Antikörpern zu definieren, welche die Serodiagnostik dieser humanpathogenen Keime entscheidend vereinfachen und verbessern. Diese Serodiagnostik kann derzeit nur über die O-spezifische Kette, mit den bekannten Nachteilen fehlender Kreuzspezifitäten, durchgeführt werden.

Die beim erfindungsgemäßen Verfahren eingesetzten Pseudomonaden, welche im Boden, Wasser, Abwasser, auf Pflanzen und in Nahrungsmitteln vorkommen, sind bestens bekannte Mikroorganismen, welche auch von anerkannten Hinterlegungsstellen erhalten werden können.

In Abhängigkeit der angewandten Bedingungen bei der Methanolyse, gelingt es mittels des erfindungsgemäßen Verfahrens, entweder das Heptose-Monosaccharid oder das Heptose-Di- bzw. Oligosaccharid herzustellen (siehe auch nachfolgendes Beispiel, 3.1).

### Kurze Beschreibung der Zeichnungen

**Fig. 1** zeigt die Strukturformel, das Elektronenstoß-Massenspektrum (1) und das Cl-[NH₃]-Massenspektrum (2) von Methyl-2,3,4,6,7 penta-O-azetyl-D-glycero-α/β-D-manno-Heptopyranose (Verbindung Nr. 4).

**Fig. 2** zeigt die Strukturformel, das Elektronenstoß-Massenspektrum (1) und das Cl-[NH₃]-Massenspektrum (2) von Methyl-2,3,4,6-tetra-O-azetyl-7-O-Carbamoyl-D-glycero-α/β-D-manno-Heptopyranose (Verbindung Nr. 5).

**Fig. 3** zeigt die Strukturformel, das Elektronenstoß-Massenspektrum (1) und das Cl-[NH₃]-Massenspektrum (2) von Methyl-7-O-(N,N-dimethylcarbamoyl)-2.3,4,6-tetra-O-methyl-D-glycero-α/β-D-manno-Heptopyranose (Verbindung Nr. 3).

**Fig. 4** zeigt die Strukturformel, das Elektronenstoß-Massenspektrum (1) und das Cl-[NH₃]-Massenspektrum (2) von Methyl-3-O-[7-O-(N,N-dimethylcarbamoyl)-2,3,4,6-tetra-O-methyl-heptopyranosyl]-2,4,6,7-tetra-O-methyl-heptopyranisid (Verbindung Nr. 12).

**Fig. 5** zeigt die Strukturformel und das Elektronenstoß-Massenspektrum von Methyl-7-O-[N,N-di-(trideuteriomethyl)-carbamoyl)-2,3,4,6-tetra-O-trideuteriomethyl-D-glycero-α/β-D-manno-Heptopyranose (Verbindung Nr. 6).

**Fig. 6** zeigt die Strukturformel, das Elektronenstoß-Massenspektrum (1) und das Cl-[NH₃]-Massenspektrum (2) von Methyl-7-O-Azetyl-2,3,4,6-tetra-O-methyl-D-glycero-α/β-D-manno-Heptopyranose (Verbindung Nr. 8).

**Fig. 7** zeigt die Strukturformel, das Elektronenstoß-Massenspektrum (1) und das Cl-[NH₃]-Massenspektrum (2) von Methyl-3-O-7-O-Azetyl-2,3,4,6-tetra-O-methyl-heptopyranosyl]-2,4,6,7,tetra-O-methyl-heptopyranisid (Verbindung Nr. 14).

**Fig. 8** zeigt die Strukturformel, das Elektronenstoß-Massenspektrum (1) und das Cl-[NH₃]-massenspektrum (2) von 1,5-Di-O-Azetyl-7-O-(N,N-dimethylcarbamoyl)-2,3,4,6-tetra-O-methyl-Heptitol (Verbindung Nr. 16).

**Fig. 9** zeigt die Strukturformel und das Elektronenstoß-Massenspektrum von 1,5,7-Tri-O-Azetyl-2,3,4,6-tetra-O-methyl-Heptitol (Verbindung Nr. 15).

**Fig. 10** zeigt die Strukturformel, das Elektronenstoß-Massenspektrum (1) und das Cl-[NH₃]-Massenspektrum (2) von 1,3,5-Tri-O-Azetyl-2,4,6,7-tetra-O-methyl-Heptitol (Verbindung Nr. 18).

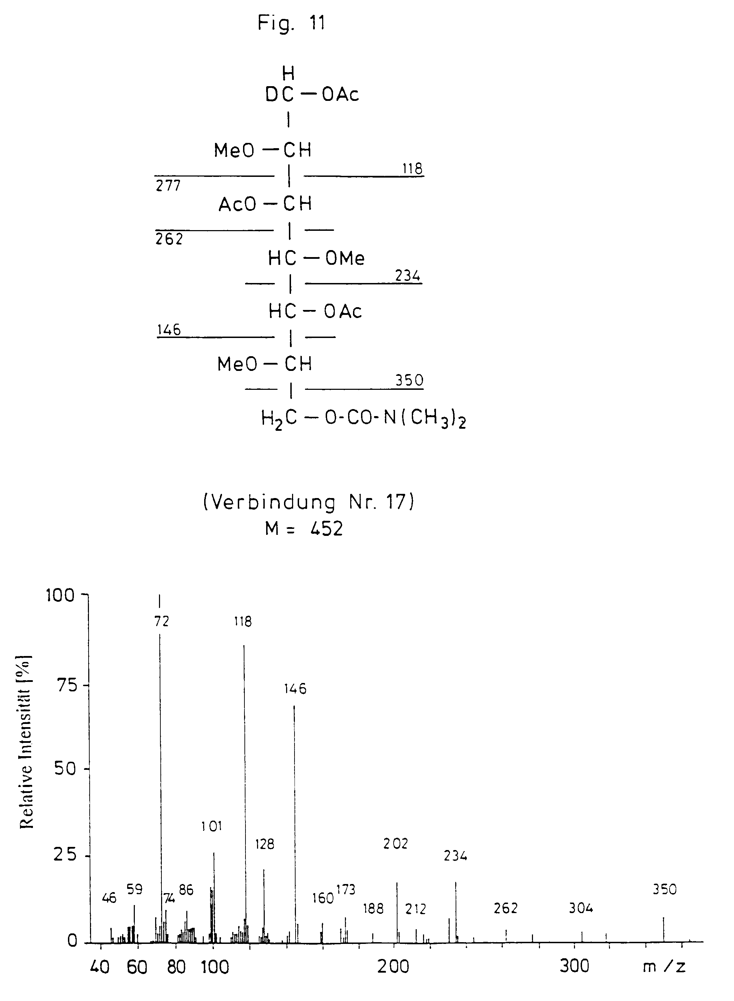

**Fig. 11** zeigt die Strukturformel und das Elektronenstoß-Massenspektrum von 1,3,5-Tri-O-Azetyl-7-O-(N,N-dimethylcarbamoyl)-2,4,6-tri-Omethyl-Heptitol (Verbindung Nr. 17).

**Fig. 12** zeigt das Gas-Flüssigkeitschromatogramm (GC) eines Standards aus per-O-azetyliertem D-glycero-D-manno-Heptitol (D,D-Hep) und L-glycero-D-manno-Heptitol (L,D-Hep) (oben) verglichen mit dem aus dem Kern-Oligosaccharid von PAC605 isolierten Heptitol-Azetaten aus Hep I und Hep II, nach Entfernung der 7-O-Carbamoyl-Gruppe.
Säule: SPB-5^{TM}, 150°C-3min dann 5°/min bis 330°C.

**Fig. 13** zeigt die Strukturformel und das ¹H-NMR-Spektrum des Methyl-7-O-Carbamoyl-LαD-Hep*p*-(1→3)-LαD-Hep*p*-(1→OMe) (Verbindung Nr. 9α) (360 MHz. D₂O, Raumtemperatur)

**Fig. 14** zeigt die Strukturformel und das ¹³C-NMR-Spektrum des Methyl-7-O-Carbamoyl-LαD-Hep*p*-(1→3)-LαD-Hep*p*-(1→OMe) (Verbindung Nr. 9α) (90 MHz. D₂O, Raumtemperatur).

**Fig. 15** zeigt die Strukturformel, die Summenformel und das Laserdesorptions-Massenspektrum des Methyl-7-O-Carbamoyl-LαD-Hepp-(1→3)-LαD-Hep*p*-(1→OMe) (Verbindung Nr. 9α).

### Beispiel

Die nachfolgend erwähnten Verbindungen besitzen die folgenden Strukturformeln, worin Me = Methyl und Ac = Azetyl.

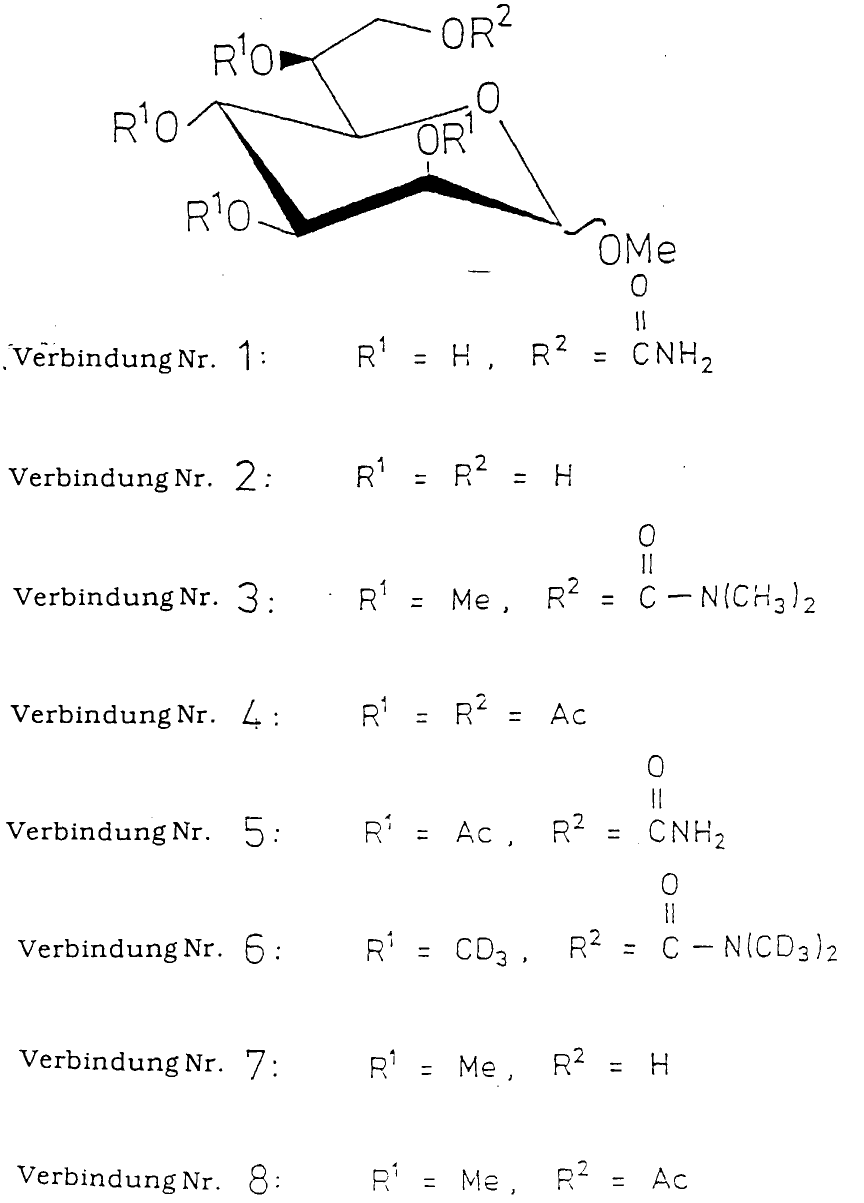

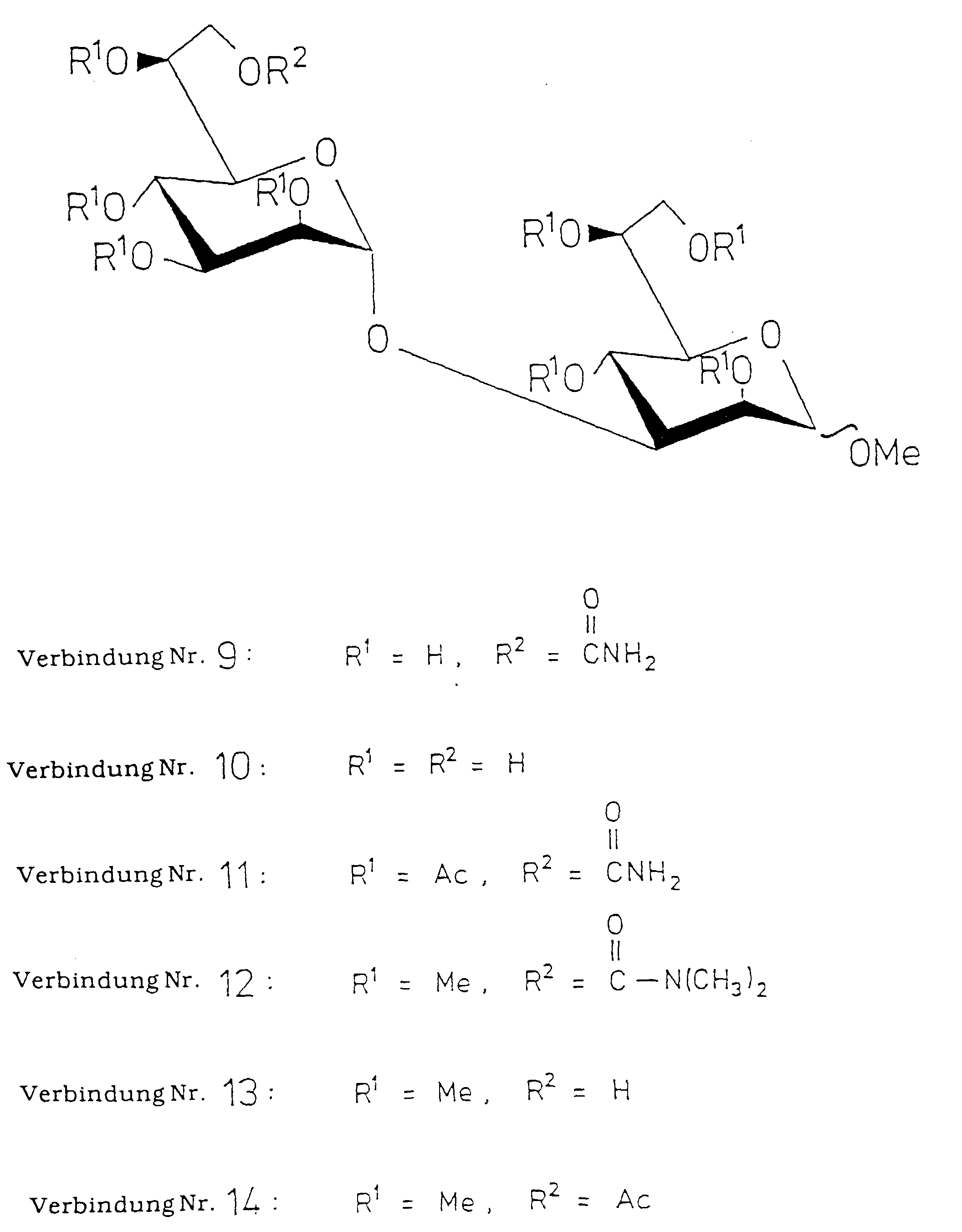

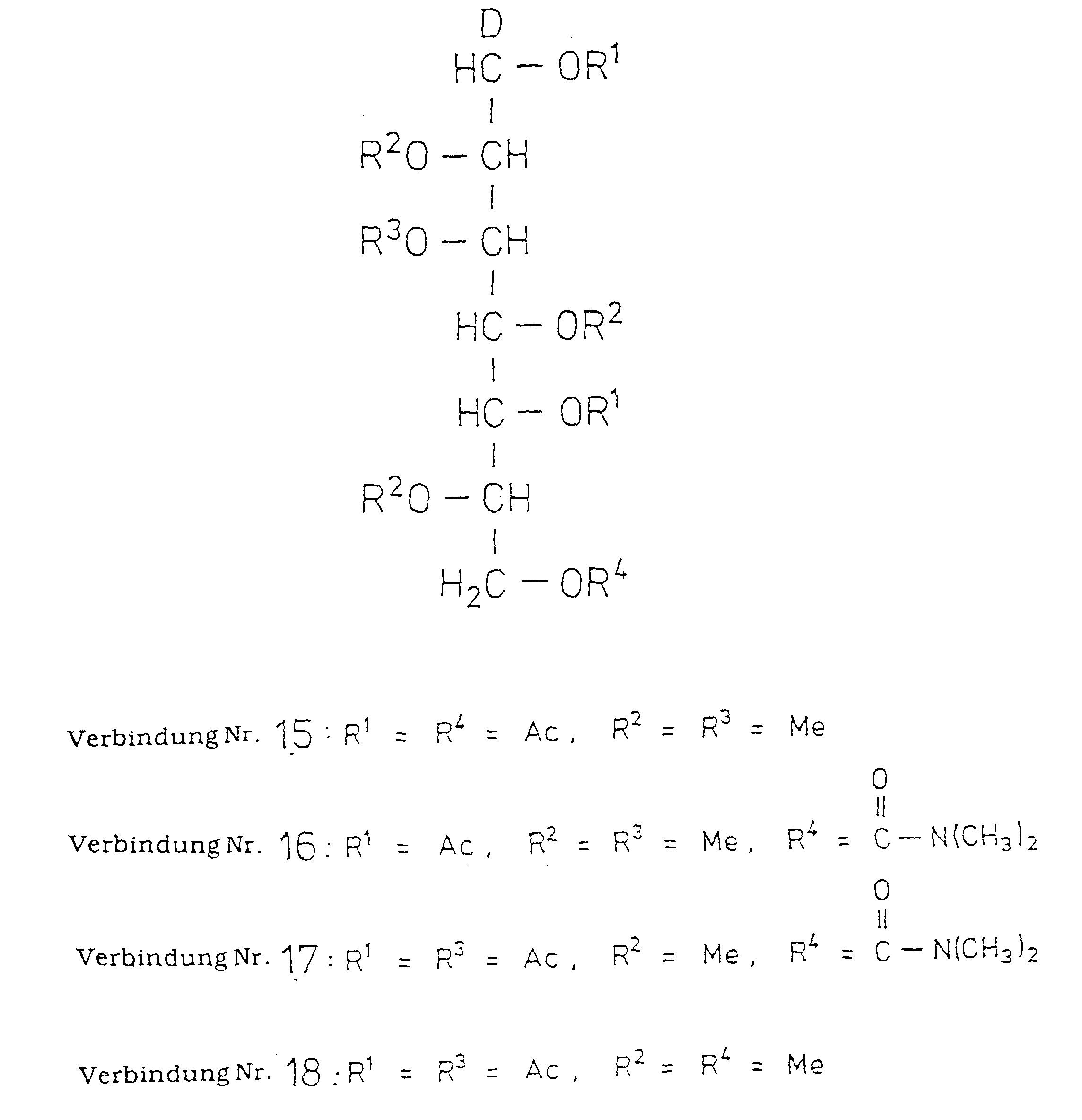

### Materialien und Methoden

### Anzucht der Bakterien und Extraktion des Lipopolysaccharids

*Pseudomonas aerigunosa-*Bakterien (PAO, PAC605, PAC557 und R5 (Habs 06)) wurden von P. Meadow, University College, London, Großbritannien [Rowe, P.S.N. und Meadow, P.M., *Eur.J.Biochem., 132,* (1983) 329-337] bzw. von J.S. Lam, University of Guelph, Ontario, Kanada, erhalten [E. Altman, et al., *Biochemistry* 1994, submitted, E. Altman et al., Int. Carb. Conference. Paris 1992, E. Altman et al., 2nd IES Conference Vienna 1992]. Die Mutante PAO ist bei der Deutschen Sammlung von Mikroorganismen und Zellkulturen GmbH. Braunschweig unter der Nummer DSM 1707 hinterlegt. Die Rauhform-Mutante PAC 605 wurde in einem 100 l Fermenter, die anderen *P*. *aeruginosa* Mutanten in 2 l Schüttelkolbenkulturen angezüchtet, wie beschrieben [Kulshin, V.A. et al.. *Eur.J. Biochem, 198,* (1991) 697-704]. Lipopolysaccharide der Stämme *P. aeruginosa* Fischer Immunotypen 2 und 7 waren von Prof. B. Dmitriev, Moskau, *P. aeruginosa* 170519, 170520 und FH-N-845 von Prof. E.S. Stanislavsky, Moskau und *Pseudomonas plantarii* DSM 7128 stammte von der DSM-Deutsche Sammlung von Mikroorganismen und Zellkulturen GmbH, Braunschweig und *P. fluorescens* ATCC 49271 wurde von der American Type Culture Collection, Rockville, Maryland, USA erhalten. Alle anderen LPS Cargen: *Klebsiella pneumoniae,* K 25: *Yersinia enterocolitica*. Mutante 490 M; *Campylobacter jejuni* RN16 O:58. CCUG 10936; *Proteus mirabilis*, Mutante R₄₅; *Haemophilus influenzae,* B, Stamm Eagan, *Vibrio parahaemolyticus,* Serotyp O12, *Salmonella minnesota,* SF 1111 (S-Form), sowie *E*. *coli* O111 (S-Form) stammten aus der LPS-Sammlung des Forschungsinstitutes Borstel.

Zur Darstellung der erwünschten Heptose als Mono- oder Oligomer wurde die *P. aeruginosa* PAC605 Mutante verwendet. Hierzu wurden getrocknete Bakterien (38.1 g) mit je 1 Liter Ethanol, Aceton und Diethylether gewaschen und das etherische Sediment getrocknet (28,6 g. 75 %). Mit der so erhaltenen Membranfraktion wurde ein Enzymverdau mit DNAse (aus Rinderbauchspeicheldrüse, Boehringer, Mannheim) und RNAse (Rinderbauchspeicheldrüse, Sigma) und Proteinase K (aus *Tritriachium album,* Boehringer) durchgeführt. Die Extraktion erfolgte nach einer modifizierten Phenol-Chloroform-Petroleum-Methode [PCP II (5 Teile Phenol, 5 Teile Chloroform, 8 Teile Petroleumether (Kp. 90°- 100°C) modifiziert nach Galanos et al., [Galanos, C., Lüderitz, O, und Westphal, O., *Eur.J. Biochem., 9,* (1969), 245-249]. Anschließend wurde ausgiebig gegen destilliertes Wasser dialysiert. Die Ausbeute betrug 2,2 g (6 %, m/m). Die Extraktion aller anderen Pseudomonas-Stämme erfolgte in analoger Weise nach der Methode von Galanos et al., mit Ausbeuten zwischen 1 und 5 %.

### Gas-Chromatographie (GC)

Die gaschromatographische Analyse wurde mit einem Hewlett Packard Gaschromatographen (Modell 5890, Series II), ausgestattet mit einem Flammenionisationsdetektor (FID), durchgeführt. Als Trägergas wurde Wasserstoff mit einem Säulenvordruck von 0.08 MPa verwendet. Als Trennsäulen diente eine SPB-5^{TM} Säule (30 m. 0,25 mm ID. 0,25 µm Filmdicke, Supelco) welche mit einem Temperaturgradienten (150°C für 3 min, dann 5 °C/min bis 330°C) betrieben wurde. Für die Auswertung wurde Hewlett Packard Chemstation Software® auf einer Vectra 486/66U benutzt.

### Kombinierte Gas-Flüssigkeitschromatographie/Massenspektroskopie (GC-MS) Analyse

Die kombinierte Gas-Flüssigkeitschromatographie/Massenspektroskopie (GC-MS) wurde mit einer Hewlett Packard HP 5989A MS Engine durchgeführt, die mit einem Gaschromatograph HP 5890 Serie II und einer Kapillarsäule (HP-5®, 30 m, 0,25 mm, 0.25 µ, Hewlett Packard) ausgerüstet war. Elektronenstoß-Massenspektren (EI-MS) wurden mit 70 eV aufgenommen. Als Reaktorgas für die chemische Ionisations-Massenspektrometrie (CI-MS) diente Ammoniak. Die Auswertung der Spektren erfolgte mit einer Hewlett Packard Chemstation Software® auf einer Vectra 486/66U.

### NMR-Spektroskopie

¹H- (360 MHz) und ¹³C- (90 MHz) NMR Spektren der Mono- und Disaccharide wurden mittels eines Bruker NMR-Spektrometers (Modell AM-360) bei Raumtemperatur in D₂O durchgeführt. Die Aufnahme und Prozessierung von eindimensionalen, homo- (¹H, ¹H-COSY) und heteronuklear (¹H,¹³C-NMR) korrelierten Daten und Spektren erfolgte mit einem ASPECT 3000 Rechner (Bruker) mit der Standard Bruker Software DISNMIR Version 89 11 01.0.

### Laser-Desorptions-Massenspektrometrie (LD-MS)

Die Laser-Desorptions-Massenspektrometrie (LD-MS) wurde mittels eines Lamma 500 Instruments (Leybold-Heraeus, Köln) im positiven Mode ohne Zusatz von kationisierenden Salzen, wie beschrieben, durchgeführt [B. Lindner et al.., *in*: *Analytical Microbiology Methods.* (1990), Plenum Press New York, A. Fox, S.L. Morgan, L. Larsson und G. Odhan (eds.), p. 149-161].

### Hochdruck-Flüssigkeitschromatographie (HPLC)

Die Hochdruck-Flüssigkeitschromatographie (HPLC) der Methyl-Heptopyranose-Mono- und Dimeren (2α, 9α und 10α) wurde an einer DuPont Anlage (Pumpe 870, Gradientencontroler 8800) an einer Zorbax-NH₂-Säule (9 x 250 mm) durchgeführt. Die Säule wurde mit einem CH₃CN-H₂O-Gradienten und einer Flußrate von 3,5 ml/min betrieben. Der Gradient zur Isolation von 9α setzte sich zusammen aus Laufmittel A [CH₃CN-H₂O 925:75 (v/v)] und Laufmittel B [CH₃CN-H₂O 75:925 (v/v)]; 0%B-5min-10%B-75min-100%B-20min-100%B]. Der Gradient für die Reindarstellung von 10α war [A: 92:8, (v/v)] und [B: 50:50, (v/v)]; 0%B-5min-0%B-75min-100%B-20min-100%B]. Das Eluat wurde mittels eines Chiraldetektors (Nr. 1000A, Knauer) auf Zuckerkomponenten detektiert und Fraktionen (3,5 ml) gesammelt (Foxy, Colora). Aliquots dieser Fraktionen wurden zusätzlich mittels Dünnschichtchromatographie (TLC) getestet. Die Retentionszeiten waren unter den genannten Bedingungen 21 min (9α) und 40 min (2α) bzw. 99 min (10α).

### Dünnschichtanalyse (TLC)

Die Dünnschichtanalyse erfolgte auf Kieselgel 60 F₂₄₅ (Merck) Aluminiumplatten in Chloroform-Methanol-Wasser (100:100:30, v/v). Die Detektion der Zuckerderivate erfolgte durch Besprühen mit 15 % (v/v) H₂SO₄ in EtOH und anschließendem Erhitzen.

### Derivatisierungs- und Abbaureaktionen

### 1. Dephosphorylierung des LPS

LPS (20 mg) wurde über Nacht mit wässr. HF (48 %) bei 4°C im Teflongefäß gerührt. Anschließend wurde ausgiebig gegen Wasser dialysiert, bis ein neutraler pH-Wert erreicht war. Das Innendialysat wurde lyophilisiert (12,2 mg, 61 % m/m).

### 2. Freisetzung des dephosphorylierten Kern-Oligosaccharids

Das dephosphorylierte LPS (LPS-HF) wurde in einer Natriumazetat-Puffer-lösung (0,1 M NaOAc, pH 4,4) 8h bei Raumtemperatur gerührt. Das dephosphorylierte Kern-Oligosaccharid konnte nach Abzentrifugieren (20.000xg, 30min) des Lipoid A und anschließender Sephadex G-10 Chromatographie (2,5 x 120 cm) isoliert werden (7,2 mg, 59 %, m/m, bezogen auf LPS-HF).

### 3. Herstellung und Derivatisierung der Methylglykoside der Heptose-Mono- und Oligomere

### 3.1. Herstellung der Heptose-Methylglykoside

Das dephosphorylierte LPS (42 mg) wurde durch Behandlung mit HCl(Me-OH in die Methylglykosid-Mono- und Dimere überführt. Zur Herstellung der Monomere wurde für 30 min bei 85°C in 2 M HCl/MeOH, zur Herstellung der Oligomere wurde für 30 min bei 85°C in 0,5 M HCl/MeOH hydrolysiert und mittels HPLC (Zorbax-NH₂) isoliert. Ausbeute an Monomer (1, 1,4 mg) und Dimer (9α. 2,1 mg)

### 3.2. Derivatisierung der Heptose-Methylglykoside

### 3.2.1 Perazetylierung

Die nach 3,1 hergestellten Methylglykoside (1.5 mg) wurden mit Azetanhydrid/Pyridin (Ac₂O/Pyridin) bei 37°C für 30 min derivatisiert und anschließend mittels GC-MS analysiert. Zur Herstellung der Deuteriummarkierten perazetylierten Heptosederivate wurde in analoger Weise mit (CD₃CO)₂/Pyridin derivatisiert.

### 3.2.2. Permethylierung

Die Methylierung der Heptosen bzw. des dephosphorylierten Kern-Oligosaccharides (je 2 mg) erfolgte nach der Methode von Ciucanu und Kerek [Ciucanu, C. und Kerek, F., *Carbohydr. Res., 131,* (1984) 209-217). Die permethylierten Heptosederivate oder Oligosaccharide wurden über Kieselgelsäule (Kieselgel 60, 70-230 mesh, 0,5 x 5 cm) gereinigt. Hierzu wurden die Säulen mit Chloroform equilibriert und mit steigender Konzentration Methanol (M) in Chloroform (C) eluiert (C-M 95:5; v/v). Zur Herstellung der trideuterio-markierten permethylierten Heptosederivate wurde in analoger Weise mit Trideuterio-Jodmethan (CD₃I) anstelle von Jodmethan derivatisiert. Für die Perethylierung der Heptose wurde Ethyljodid anstelle von Jodmethan verwendet.

### 3.2.3. Reduktive Spaltung des Dimethyl-Carbamoylrestes mittels Lithiumaluminiumhydrid

Systematische Voruntersuchungen an der permethylierten 7-O-Carbamoyl-Heptose ergaben, daß der 7-O-Carbamoyl-Substituent nach Dephosphorylierung (48 % HF), NaOAC-Hydrolyse, Methanolyse und Permethylierung als 7-O-Carbamoyl-N,N-Dimethyl-Rest an der Heptose erhalten bleibt. Für die Freisetzung des 7-O-Carbamoyl-Heptose wurden je 2 mg des permethylierten 7-O-Carbamoyl-N,N,Dimethyl-Heptose-Monomers 3 oder Dimers 12 in trockenem Diethylether (1 ml) gelöst und mit 7,5 mg Lithiumaluminiumhydrid (LiAIH₄) für 30 min bei Raumtemperatur gerührt. Das Lösungsmittel wurde im Stickstoffstrom abgeblasen und überschüssiges LiAlH₄ durch tropfenweise Zugabe von Wasser zerstört. Zur weiteren Reinigung wurde das Produkt in Chloroform-Methanol (98:2, v/v) aufgenommen, das überschüssige Salz mittels Zentrifugation abgetrennt und getrocknet (Ausbeuten: 7, 0,9 mg und 13, 0,7 mg).

### 3.2.4. Regioselektive Resubstitution des decarbamoylierten Heptose-Mono- und Dimers

Die Heptosederivate 7 und 13 wurden mittels N,N-Dimethylcarbamoylchlorid (50 µl, SIGMA) in Pyridin (500 µl) für 4h bei 85°C erhitzt. Anschließend wurden Lösungsmittel und Reagenz im Ölpumpenvakuum entfernt und die Produkte massenspektrometrisch untersucht. Alternativ zur Resubstitution mittels N,N-Dimethylcarbamoylchlorid, wurde mit Azetanhydrid/Pyridin derivatisiert (3.2.1.) und die erhaltenen Produkte (8 und 14) mittels GLC-MS untersucht.

### 3.2.5. Methylierungsanalyse der Heptose zur Bestimmung der Bindungsverhältnisse in der Heptoseregion

Die Methylierungsanalyse diente zunächst zur Bestimmung des Substitutionsortes des Carbamoylrestes. Hierzu wurden Monosaccharid 3, Disaccharid 12 und permethyliertes Oligosaccharid zunächst mit 0.5 ml 2 M Trifluoressigsäure (TFA, Merck) 1 h bei 120°C hydrolysiert. Unter diesen Bedingungen wird der N,N-Dimethyl-7-O-Carbamoyl-Rest vollständig abgespalten. Überschüssige TFA wurde durch dreifaches Abdampfen mit destilliertem Wasser am Rotationsverdampfer abgezogen. Anschließend wird die Probe mit Natriumbordeuterid (NaBD₄) reduziert, azetyliert und das partiell methylierte deutero-reduzierte Heptitol-Azetat mittels GC und GC-MS untersucht.

### 3.3. Zuordnung der Carbamoyl-Heptose zur L-glycero-D-manno-Heptitol- und D-glycero-D-manno-Heptitol-Konfiguration

Alle in dem LPS bislang gefundenen Heptosen gehören ausnahmslos der L-glycero-D-manno-Heptopyranose- oder D-glycero-D-manno-Heptopyranose-Konfiguration an. Um die 7-O-Carbamoyl-Heptose einer dieser Konfigurationen zuordnen zu können, wurden 4 mg des LPS zu LPS-HF dephosphoryliert. Anschließend wurde der 7-O-Carbamoyl-Substituent selektiv unter milden alkalischen Bedingungen abgespalten (0,5 M NaOH, Raumtemperatur, 30 min). Anhand von kinetischen Studien konnten wir zeigen, daß unter diesen Bedingungen der Carbamoylrest selektiv abgespalten wird, ohne daß die Heptose dabei zerstört wird. Anschließend wurde hydrolisiert (0,1 M HCl, 48h, 100°C), reduziert (5 % NaBH₄ in 10 mM NaOH, über Nacht, Raumtemperatur) und perazetyliert (Ac₂O/Pyridin). Die relative Zuweisung der Konfiguration erfolgte mittels GC-Analyse durch Vergleich mit den Retentionszeiten von Standard-L-glycero-D-manno-Heptitol und D-glycero-D-manno-Heptitol die als Diastereomerenpaare in der GLC gut unterscheidbar sind. GC-Bedingungen: Säule SPB-5® (30 m, 0.25 mm, 0,25 µ, Supelco); Gradient: 150°C für 3 min, dann 5°/min bis 330°C.

### 3.4. Screening-Verfahren zur Bestimmung der 7-O-Carbamoyl-Heptose in getrockneten Bakterien

Getrocknete Bakterien (0,2 g) werden in 0,5 ml 48 % wässr. HF (Merck) suspendiert und über Nacht in einem mit Teflonrührer versehenen Teflongefäß bei 4°C intensiv gerührt. Anschließend wurde ausgiebig gegen destilliertes Wasser dialysiert und das Retentat gefriergetrocknet. Die 7-O-Carbamoyl-Heptose wurde sodann mittels Methanolyse (0,5 M HCl/MeOH, 85°C. 30 min) aus dem LPS der Biomasse freigesetzt, methyliert [Ciucanu, C. und Kerek, F., *Carbohydr. Res., 131,* (1984) 209-217] und das permethylierte Produkt abzentrifugiert (3.000 rpm, 15 min, Rotixa, Hettlich). Anschließend wurden die Fettsäuremethylester des Lipoid A mittels einer kleinen Säule (Kieselgel 60, 70-230 mesh, 0,5 x 30 mm, Merck) in 5 ml Ether/n-Hexan (40:60, v/v) entfernt und dann das Produkt (3) mit Chloroform-Methanol (C-M 95:5, v/v) eluiert und mittels GLC-MS an einer SPB-5® Säule analysiert und quantifiziert. Die Retentionszeit (t_{R}) der permethylierten Methyl-7-O-(N,N-dimethylcarbamoyl)-L-glycero-α-D-manno-Heptopyranose anomeren Strukturen (3α und 3β) betrug unter den genannten Bedingungen (SPB-5®) 17,76 min bzw. 17,86 min.

### 3.5. Isolierung von 7-O-Carbamoyl-LαD-Hepp-(1→3)-LαD-Hepp-(1→OMe), 9α, für die NMR-Analyse

Zur Isolierung und Reindarstellung des Disaccharides 9α wurden 50 mg LPS zunächst hydrolisiert (0,1 M HCl, 100°C, 85 min) und das präzipitierende Lipoid A abzentrifugiert. Der Überstand wurde über Sephadex G-10 (2,5 x 120 cm, Wasser) weiter gereinigt und anschließend lyophilisiert. Dieses Kern-Oligosaccharid wurde in 4 Aliquots à 0,5 ml mit wässriger HF (48 %, Merck) suspendiert und in Teflongefäßen über Nacht intensiv gerührt. Nach Trocknen an der Ölpumpe (6h) wurde hydrolisiert (0,5 M HCl/MeOH, 85°C, 30 min) und das Hydrolysat über einen Ionenaustauscher (Amberlite IR-120, H⁺-Form von den aminohaltigen Komponenten GalN und Ala befreit. Nach dem Waschen (50 ml) des Austauscherharzes wurde das H₂O-Eluat über HPLC weiter aufgereinigt. Das gereinigte Disaccharid 9α, das im TLC mit einem R_{f} = 0,54 gefunden wurde, eluierte in der HPLC mit einer Retentionszeit von 21 min (Ausbeute 9α, 1,23 mg).

### 3.6. Isolierung von LαD-Hepp-(1→3)-LαD-Hepp-(1→OMe), 10α, und LαD-Hepp-(1→OMe), 2α, aus dem synthetischen Disaccharid LαD-Hepp-(1→3)-LαD-Hepp als Referenzsubstanzen für die NMR-Analyse

3,8 mg des synthetischen Disaccharides 3-O-(L-glycero-α-D-manno-Heptopyranosyl)-L-glycero-D-manno-Heptopyranose [Paulsen, H. et al., *Liebigs. Ann. Chem.*, (1986), 675-686] wurden in 0,5 M HCI/MeOH bei 37°C über Nacht inkubiert. Die TLC-Analyse [Kieselgel 60, Chloroform-Methanol-Wasser (C-M-W) 100:100:30] ergab, daß unter diesen Bedingungen neben der Zielstruktur Methyl-3-O-(7-O-Carbamoyl-L-glycero-α-D-manno-Heptopyranosyl)-L-glycero-α-D-manno-Heptopyranose-Disaccharid (10α) (R_{f} 0,40) teilweise die Monosaccharide Methyl-L-glycero-α-D-manno-Heptopyranosid 2α (R_{f} 0,58) und Methyl-*L*-glycero-β-D-manno-Heptopyranosid 2β (R_{f} 0,52) gebildet worden waren. Diese einzelnen Komponenten konnten in einem anschließenden HPLC-Lauf /Zorbax-NH₂, 9 x 250 cm, DuPont) mit einem steigenden Gradienten von Laufmittel A [CH₃CN-H₂O 92:8 (v/v)] und Laufmittel B [CH₃CN-H₂O 50:50 (v/v)] bei einem Fluß von 3,5 ml getrennt werden. Die Disaccharid-Zielstruktur 10α konnte dabei von den Monosacchariden 2α und 2β abgetrennt werden, wobei die α-anomeren Methylglycoside positive und das β-anomere Methyl-Heptose ein negatives Signal im Chiral-Detektorzeigte. Aliquots dieser Peaks wurden noch einmal mittels TLC getestet (C-M-W 100:100:30). Das gereinigte Disaccharid 9α (TLC R_{f} = 0,4) eluierte in der HPLC mit einer Retentionszeit von 99 min, 2α (40 min, TLC R_{f}0,58) und 2β (54 min, TLC R_{f}0,52). Die Ausbeuten betrugen: 10α, 0,81 mg, 2α 1,7 mg, 2β 0,70 mg.

### 4. Ergebnisse und Diskussion

Zur Charakterisierung der erfindungsgemäß erwünschten Heptose wurde zunächst die Natur und Position des Carbamoyl-Restes an der Heptose mittels verschiedener Markierungsexperimente in der GC-MS-Analyse bestimmt. Danach wurde die Verknüpfung der beiden gefundenen Heptosen im Kern-Oligosaccharid von *Pseudomonas aeruginosa* PAC605 LPS und das Substitutionsmuster des Carbamoyl-Restes im Disaccharid ebenfalls mittels GC-MS bestimmt. Schließlich konnte das Heptose-Disaccharid mit dem 7-O-Carbamoyl-Rest an der Heptose in intakter Form isoliert werden und mittels ein- und zwei-dimensionaler ¹H- und ¹³C-NMR homo-(¹H,¹H-COSY) und heteronuklear (¹H,¹³C-COSY) korrelierten NMR-Spektrometrie eingehend analysiert werden. Nachdem die 7-O-Carbamoyl-L-glycero-D-manno-Heptopyronase identifiziert war, wurde ein Screening-Verfahren zu deren Bestimmung in Bakterien entwickelt, ohne daß eine Extraktion des LPS zuvor erfolgte.

### 4.1. GC-MS-Analyse der 7-O-Carbamoyl-Heptose als per-O-azetyliertes Derivat 5

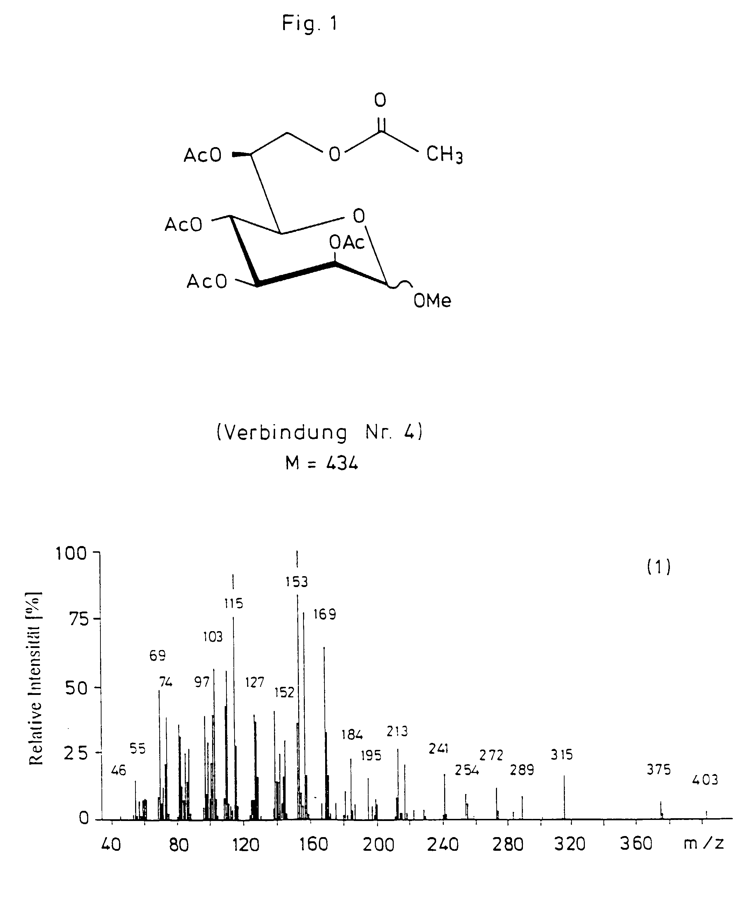

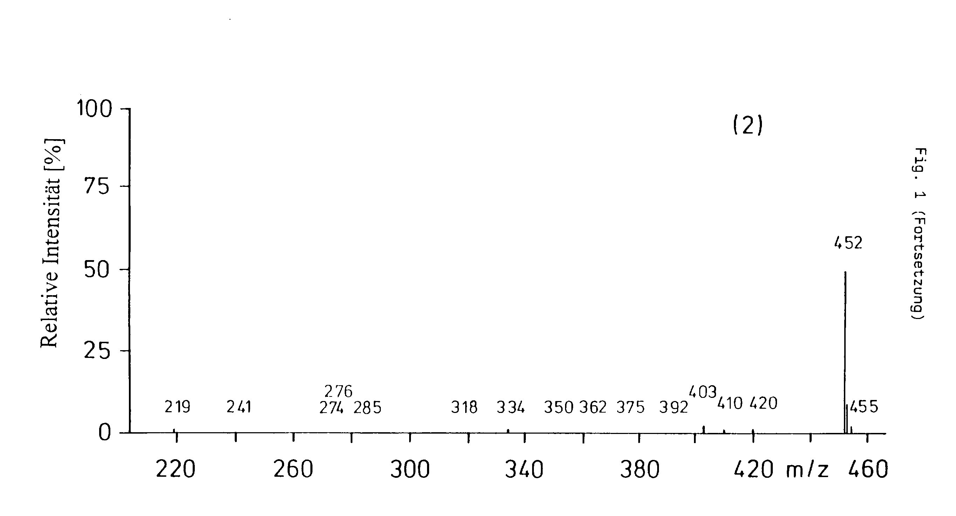

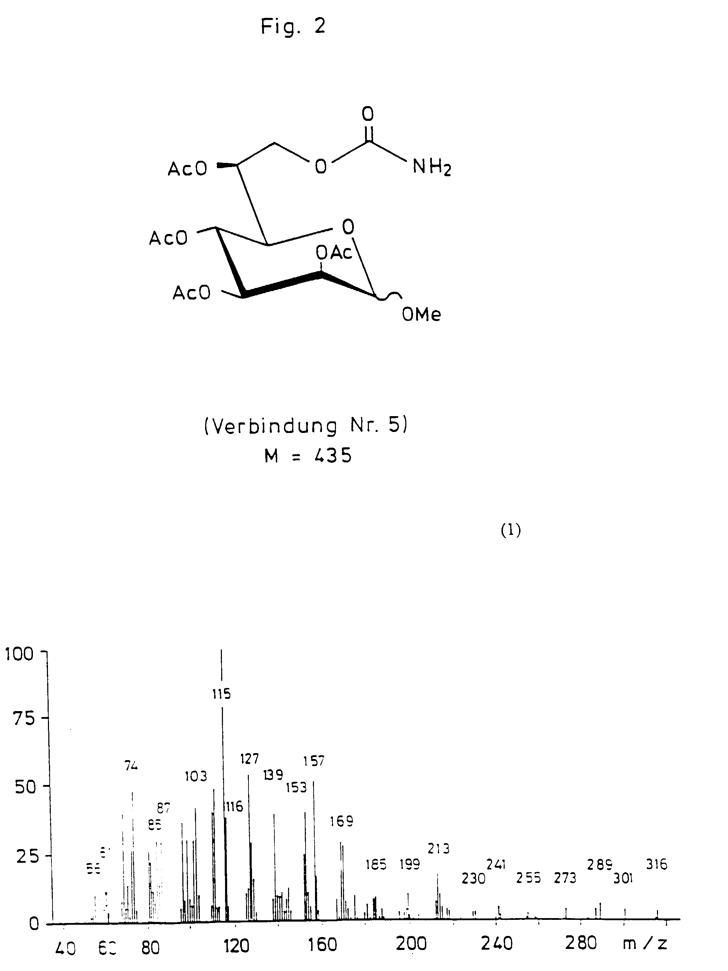

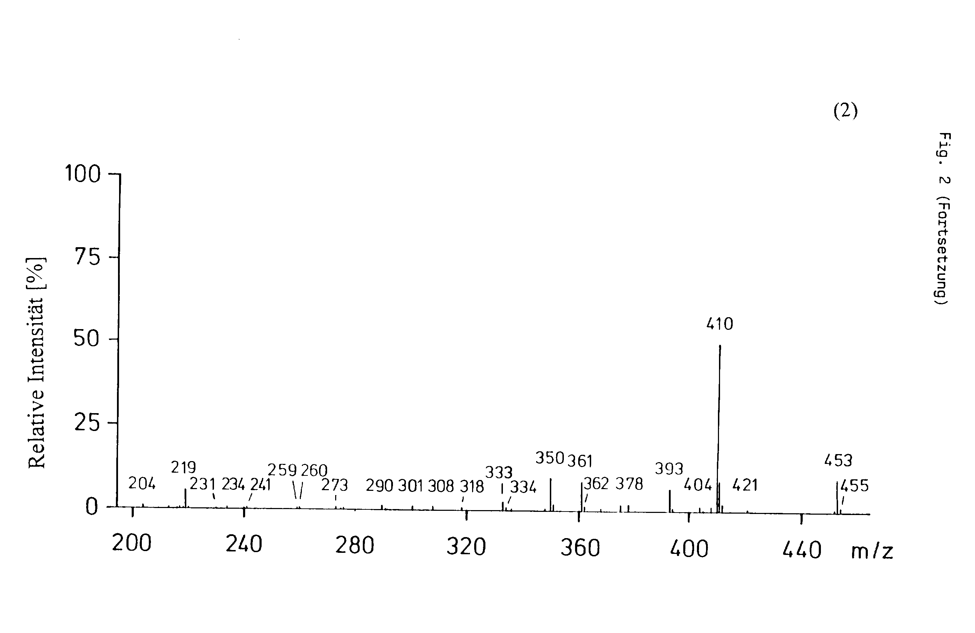

Die Methanolyse (85°C, 2 M HCl/MeOH, 30 min) am isolierten, dephosphorylierten Kern-Oligosaccharid von *P. aeruginosa* PAC605 LPS, per-O-Azetylierung und anschließende GC-MS-Analyse zeigte zwei verschiedene Heptosen. Die eine eluierte als Methyl-2,3,4,6,7-penta-O-azetyl-Heptopyranosid (4) mit einer kürzeren Retentionszeit (t_{R} = 17,4 min) als ein zweites, bisher unbekanntes Heptosederivat (t_{R} = 21,5 min). Im CI-MS zeigten beide Heptosen einen Pseudomolekülionenpeak [M + NH₄]⁺ = 452 (4) und [M + NH₄]⁺ = 453 (5) (Fig. 1 und 2). Der Massenunterschied von einer Atommasseneinheit (AMU) zwischen 4 und 5 sowie die signifikant erhöhte Retentionszeit sind durch die Substitution eines Azetylrestes (CO-CH₃) in 4 durch einen Carbamoylrest (CO-NH₂) in 5 erklärbar.

### 4.2. Identifizierung der Carbamoylgruppe mittels GC-MS

Zur Identifizierung der Carbamoylgruppe und zur Bestimmung ihres Substitutionsortes wurden verschieden markierte Derivate der unbekannten Heptose für die GC-MS-Analyse hergestellt (3 - 8), um einen Strukturbeweis mit dieser Methode führen zu können.

### 4.2.1 GC-MS-Analyse der permethylierten 7-O-Carbamoyl-Heptose als Mono- und Disaccharid

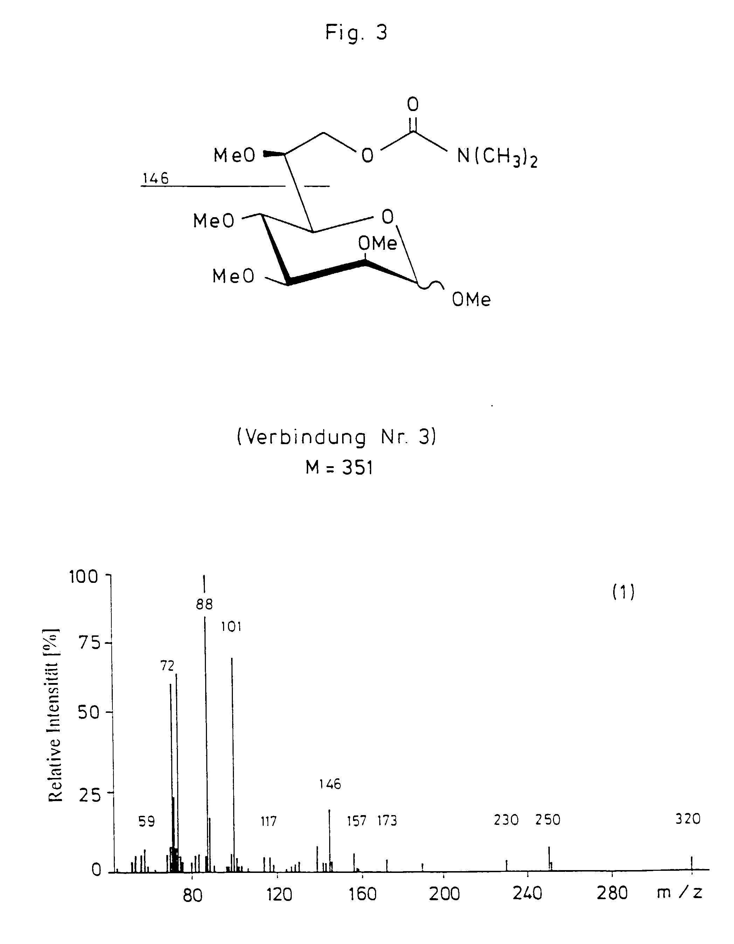

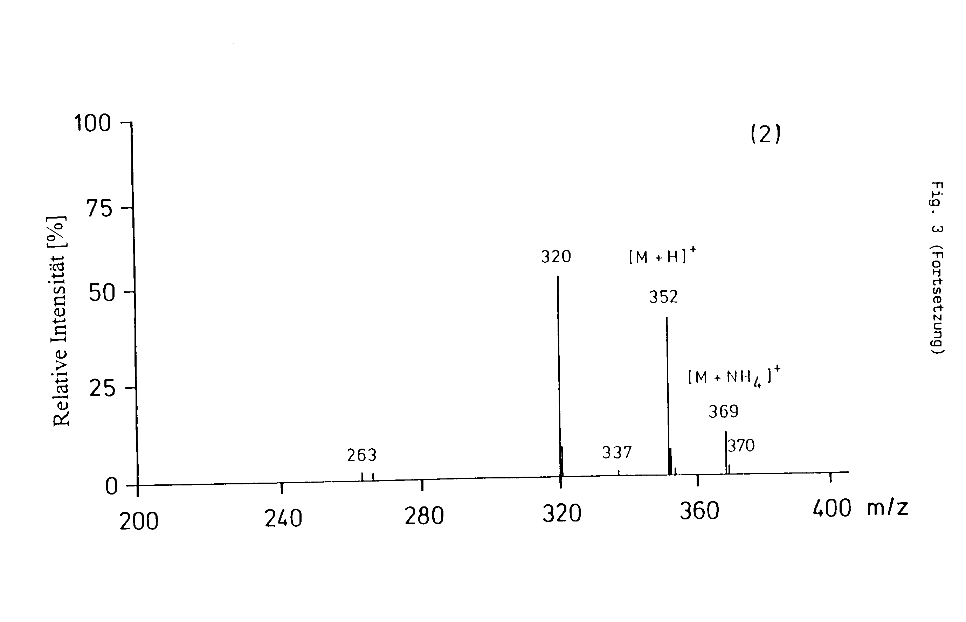

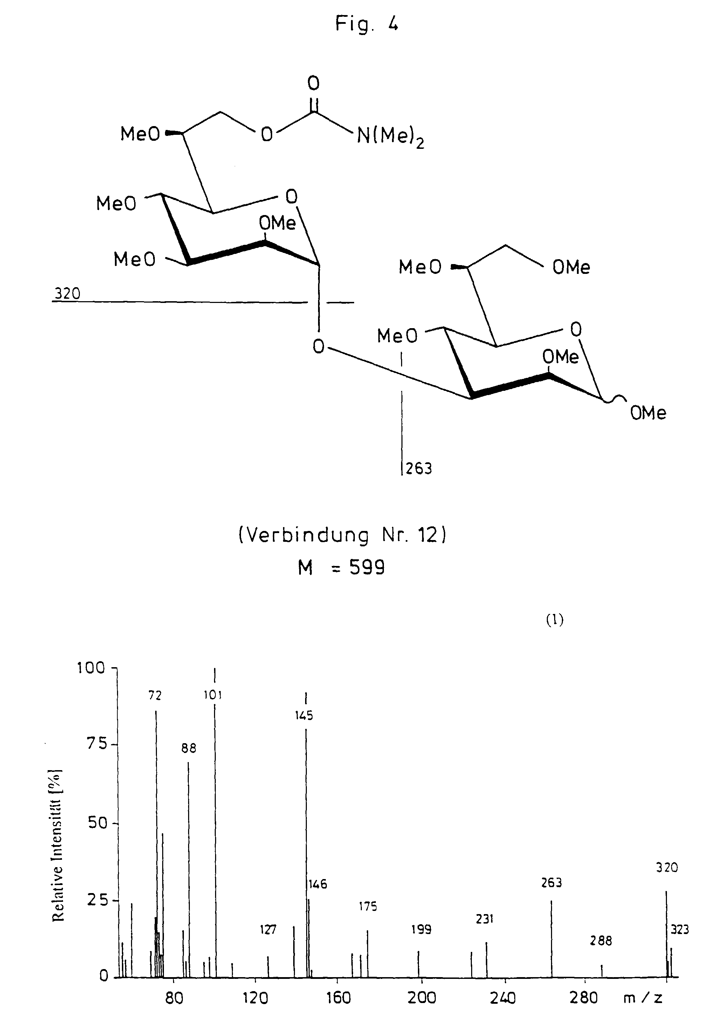

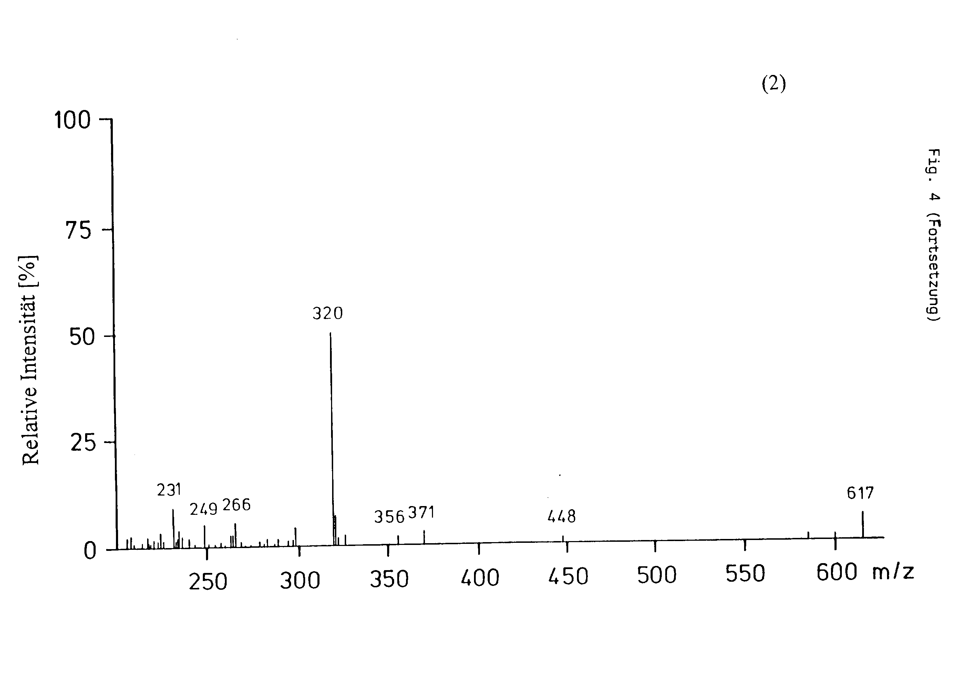

Nach Dephosphorylierung und Methanolyse wurden die Methylglykoside der Heptose permethyliert. Dabei konnte die permethylierte 7-O-Carbamoyl-Heptose sowohl als Methyl 7-O-(N,N-dimethylcarbamoyl)-2,3,4,6-tetra-O-methyl-heptopyranosid (3) als auch als Methyl 3-O-[7-O-(N,N-di-methylcarbamoyl)-2,3,4,6-tetra-O-methyl-heptopyranosyl]-2,4,6,7-tetra-O-methyl-heptopyranisid (12) in der GC-MS-Analyse identifiziert werden (Fig. 3 und Fig. 4). Dabei zeigte das Monosaccharid 3 im EI-MS ein charakteristisches Fragmention m /z = 320 [M-OCH₃]⁺ und m/z = 146 was aus dem Massenfragment [HC = O⁺Me-CO-N(CH₃)₂], welches aus der Bindungsspaltung zwischen C-5/C-6 hervorgegangen ist, erklärt werden kann. Damit konnte zunächst eine Substitution des Carbamoylrestes an C-6 bzw. C-7 wahrscheinlich gemacht werden.

Das Disaccharid 12 (t_{R} = 34,3 min) (Fig. 4) zeigte im EI-MS ein Massenfragment m/z = 320, was darauf hindeutete, daß der Carbamoylsubstituent an der zweiten, der nicht-reduzierenden Heptose (Hep II) lokalisiert sein mußte. Diese Interpretation steht im Einklang mit der Existenz eines Massenfragmentes m/z = 263, das der permethylierten, reduzierenden Heptopyranose (Hep I) zugeordnet werden konnte. Die Ergebnisse der CI-MS stützen diese Interpretation ([M + NH₄]⁺ = 617, Fig. 4). Durch Perethylierung anstelle der Permethylierung konnten Monosaccharid 3 und Disaccharid 12 in die betreffenden perethylierten Derivate überführt werden, wobei 6 bzw. 10 Ethylgruppen anstelle der Methylgruppe in 3 bzw. 12 eingeführt werden konnten, was in der MS-Analyse durch ein Masseninkrement von Δm/z = 14 AMU pro eingeführten Ethylrest nachgewiesen werden konnte (Spektren hier nicht näher dokumentiert).

### 4.2.2. Markierungsexperimente und GC-MS-Analyse am 7-O-Carbamoyl-Heptose-Mono- und Disaccharid

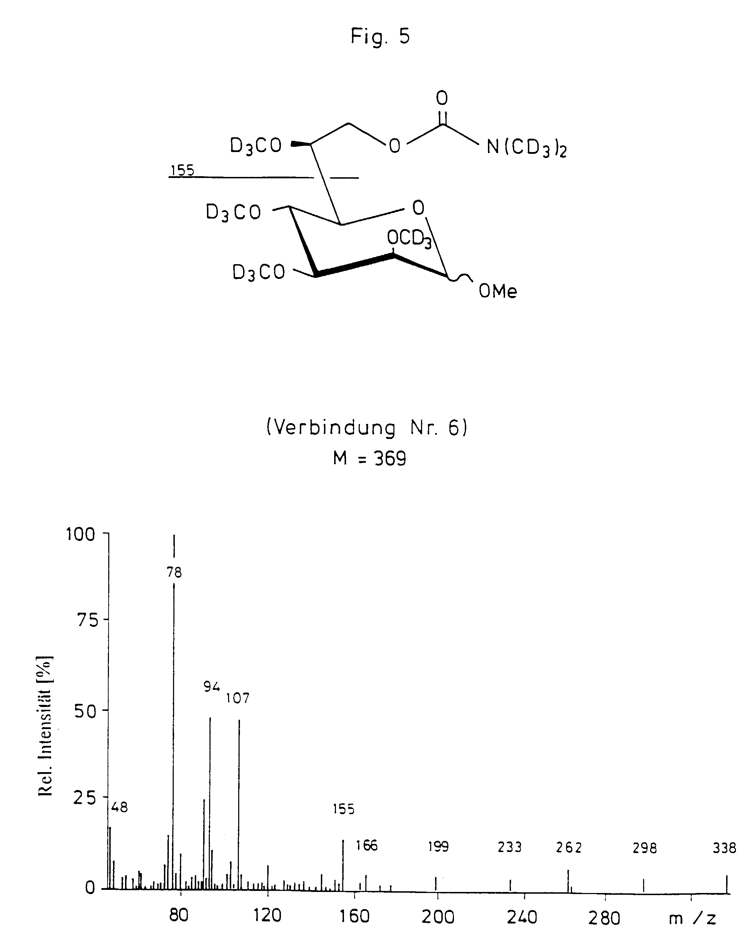

Wurde anstelle von Jodmethan mit Jodmethan-d₃ methyliert, so konnte als Monosaccharid Verbindung 6 in der GC-MS-Analyse identifiziert werden (Fig. 5). Auch hier konnte das charakteristische [M-OMe)⁺-Fragment m/z = 338 beobachtet werden, welches dem Fragment m/z = 320 in 3 entspricht. Analog fand man m /z = 155, was aus der Bindungsspaltung von C-5/C-6 hervorgegangen war. Aus dem Fragment m/z = 107 [CH=O-CO-N(CD₃)₂], welches dem nicht-diagnostischen m/z = 101 [CH=O-CO-N(CH₃)₂] in 3 entspricht, konnte die Carbamoylgruppe somit eindeutig in Position C-7 der Heptose lokalisiert werden.

### 4.2.3. Reduktive Spaltung des 7-O-Carbamoyl-Restes mittels LiAlH₄

Durch eine schonende und selektive Abspaltung des permethylierten Carbamoylrestes von 3 bzw. 12, mittels LiAlH₄ in Ether, konnten die Verbindungen 7 bzw. 13 erhalten werden, die eine freie primäre Hydroxylgruppe in Position 7 bzw. 7' anstelle der permethylierten 7-O-Carbamoylgruppe tragen. Regioselektive Resubstitution dieser freien Hydroxylgruppen mittels N,N-Dimethylcarbamoylchlorid in Pyridin lieferte die Ausgangsverbindungen 3 und 12, die sich hinsichtlich Retentionszeit und Massenfragmentierung (EI-MS, CI-MS) nicht von den Ausgangssubstanzen unterschieden. Damit konnte ein weiteres Indiz für die postulierte Struktur erhalten werden.

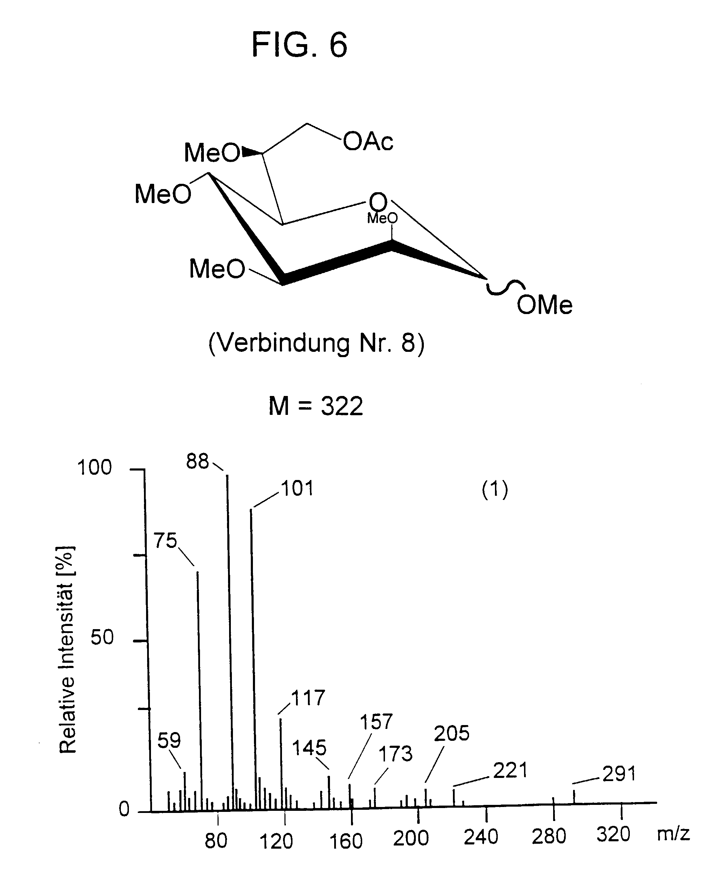

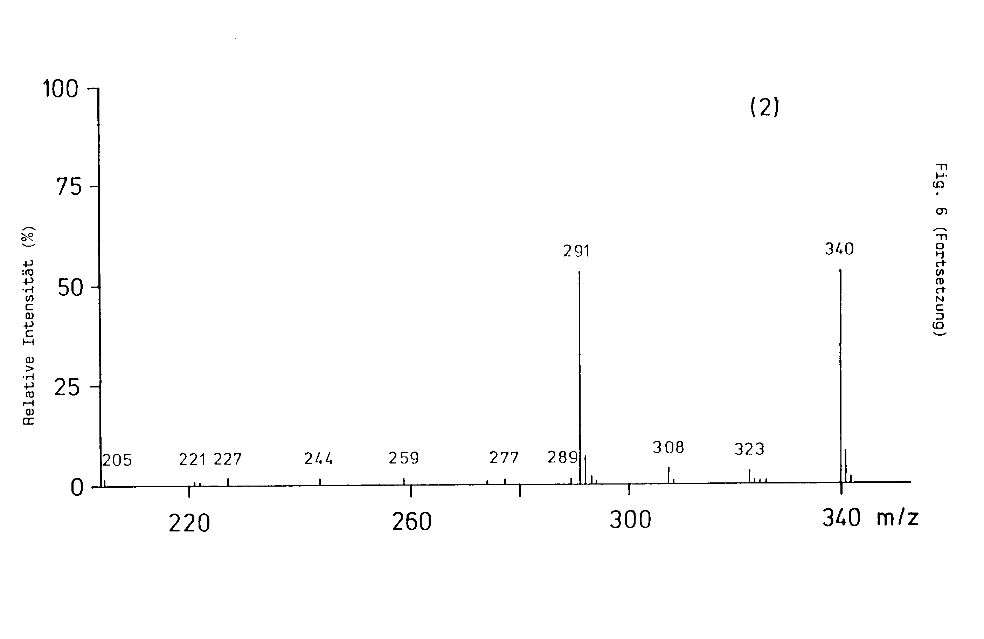

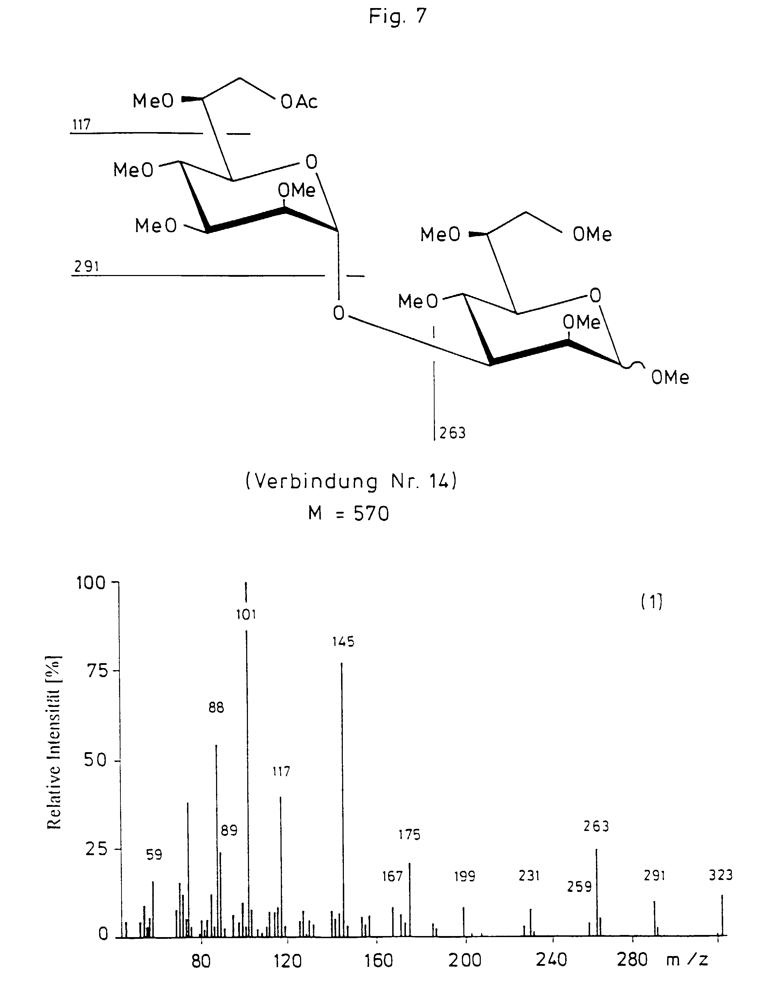

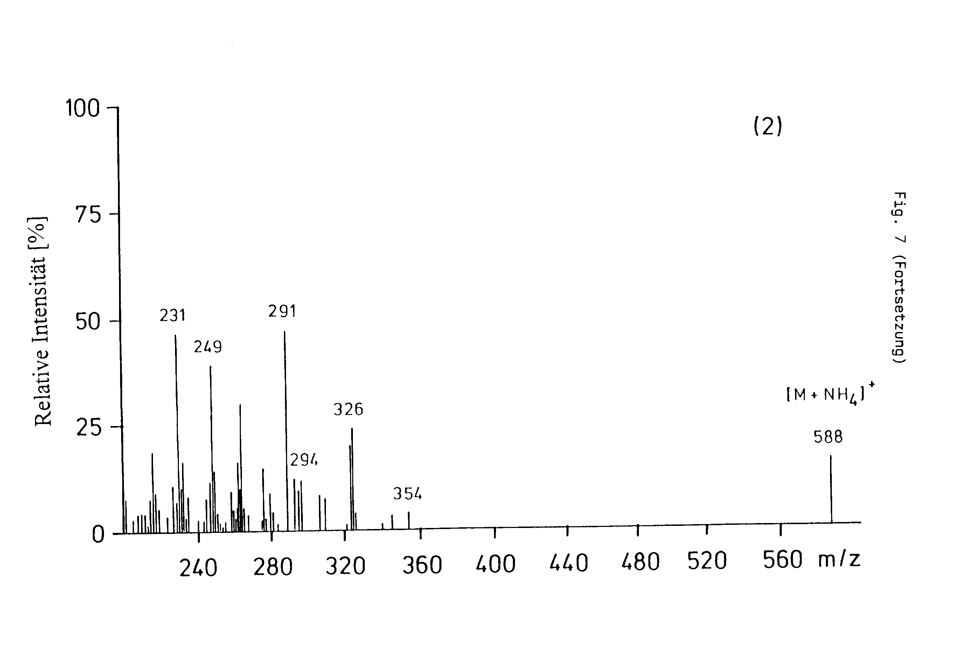

Werden 7 und 13 O-azetyliert, so erhält man 8 bzw. 14 (Fig. 6 und Fig. 7), welche beide das charakteristische Fragment m/z = 117 [CHOMe-CH₂-OAc]⁺ aufweisen, das aus der C-5/C-6 Bindungsspaltung der Heptose hervorgegangen ist. Aus der Tatsache, daß sowohl im Disaccharid 12 als auch in 14 das Massenfragment m/z = 263 nicht verändert wurde, konnte ebenfalls geschlossen werden, daß nur die terminale Heptose (Hep II) mit dem Carbamoylrest substituiert war und daß eine Substitution an Hep I ausgeschlossen ist.

### 4.3. Methylierungsanalyse der Heptose-Region im Kern-Oligosaccharid

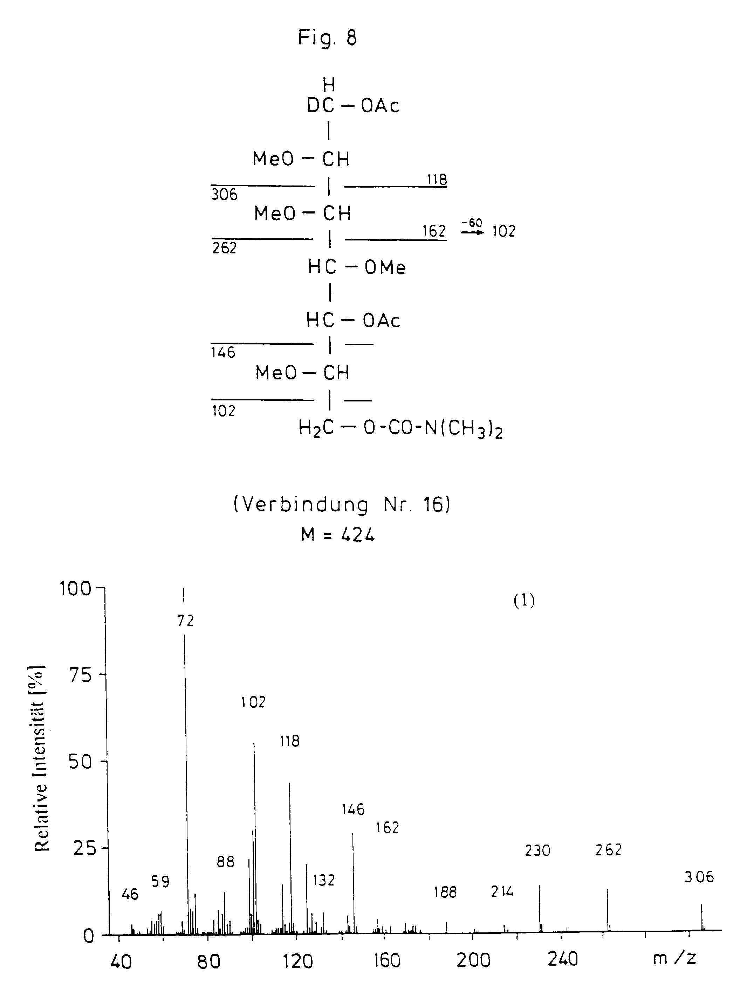

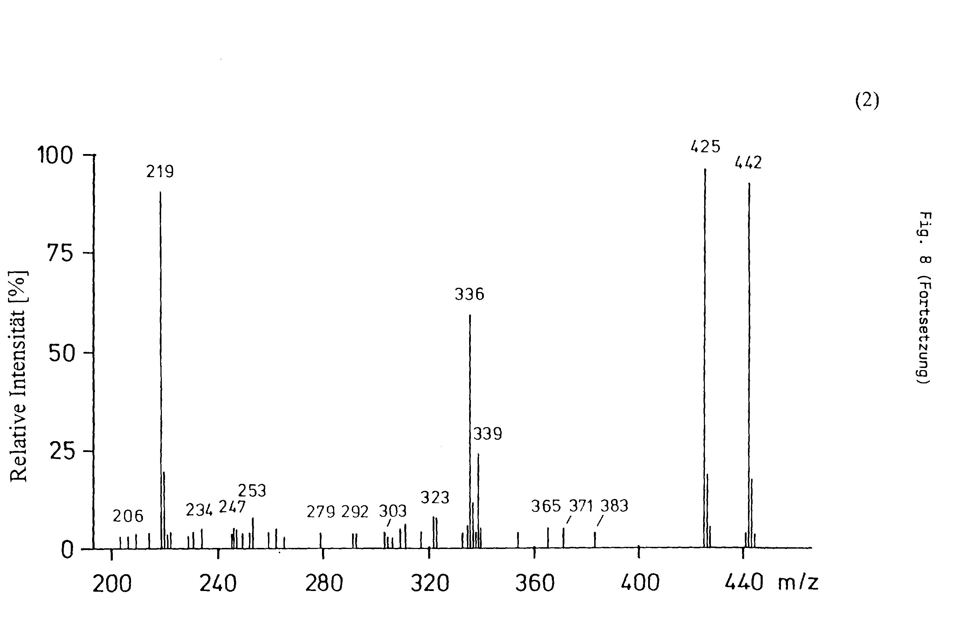

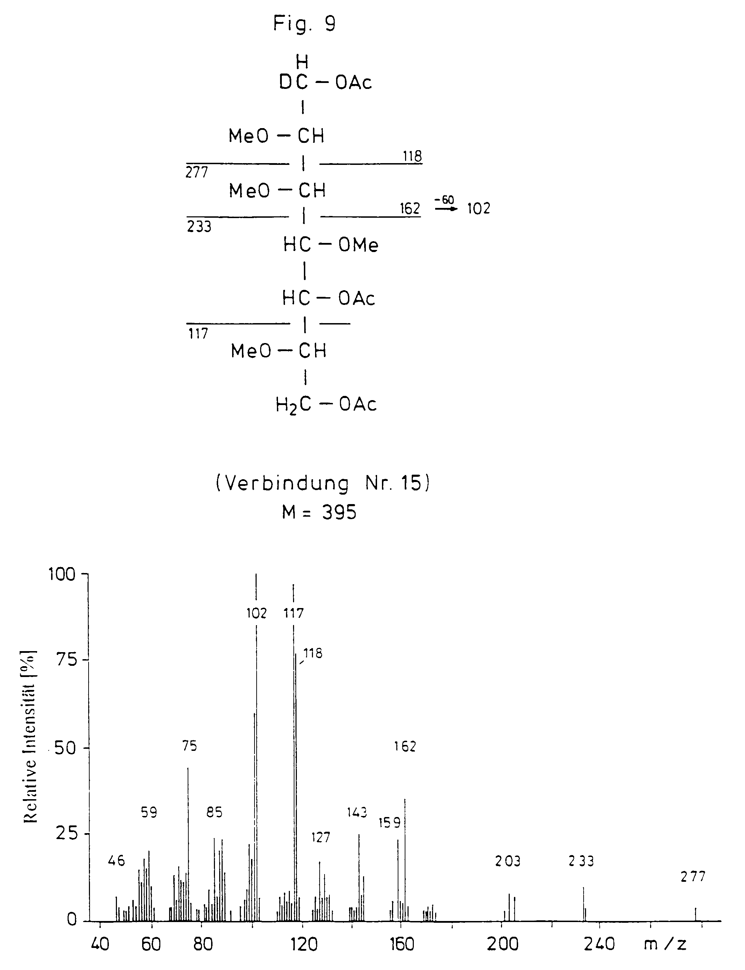

Das Monosaccharid 3 (3,5 mg) wurde hydrolysiert (2 M TFA, 1h, 120°C), reduziert (NaBD₄) und per-O-azetyliert. Wie wir fanden, wird unter diesen Bedingungen der 7-O-Carbamoyl-Rest nicht quantitativ abgespalten und es werden sowohl 1,5,7-Tri-O-azetyl-2,3,4,6-tetra-O-methyl-Heptitol, 15 (Fig. 9) als auch 1,5-Di-O-azetyl-7-O-(N,N-dimethylcarbamoyl),2,3,4,6-tetra-O-methyl-Heptitol, 16 erhalten (Fig. 8). Das partiell methylierte Heptitol-Azetat 15 (t_{R} = 15,1 min, Fig. 9) zeigte im Cl-MS [M + NH₄]⁺ m/z = 413 und [M + H]⁺ m/z = 396 und im EI-MS Fragmente m/z = 102 (162- 60), 118 und 162 vom Deuterium reduzierten Ende, sowie m/z = 117, 233, und 277 vom nicht reduzierenden Ende. Das partiell methylierte Heptitol 16 (t_{R} = 19,7 min) zeigte im Cl-MS (Fig. 8) [M + NH₄]⁺ m/z = 442 und [M + H]⁺ m/z = 425 und im EI-MS Fragmente m/z = 102 (162 - 60), 118, 162 vom Deuterium reduzierten Ende, sowie m/z = 102, 146, 262, 306 vom nicht reduzierenden Ende. Mit diesen Massenfragmenten der partiell methylierten Heptitolazetate konnte ein weiteres Indiz für die 7-Substitution des Carbamoylrestes erbracht werden.

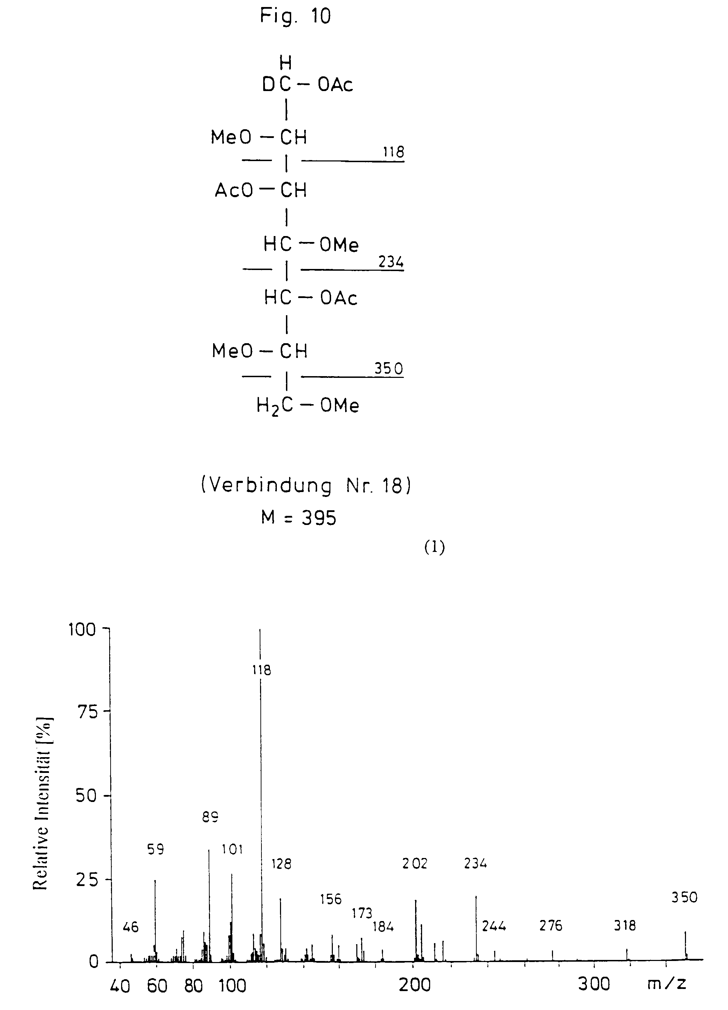

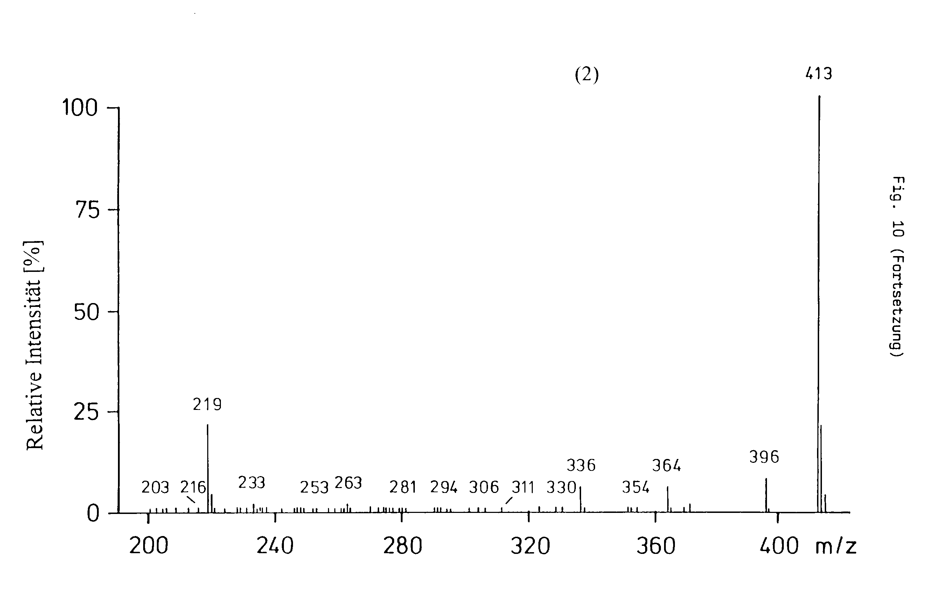

Die Verknüpfung der beiden Heptosen (Hep II - Hep I) untereinander konnte durch eine Methylierungsanalyse des permethylierten Disaccharids 12 bestimmt werden. Man erhielt 1,3,5-Tri-O-azetyl-2,4,6,7-tetra-O-methyl-Heptitol (18, t_{R} = 15,1 min, Fig. 10) woraus die Bindung der Heptosen untereinander als Hep*p*-(1→3)-Hep*p* bestimmt werden konnte. Die Massenfragmente (EI-MS) waren m/z = 118, 234, 350 und wurden als Fragmente des reduzierenden Endes identifiziert (Fig. 10). Die Daten der CI-MS-Analyse [M + NH₄]⁺ m/z = 413 und [M + H]⁺ m/z = 396 stehen mit dieser Interpretation in Einklang.

Wurde dagegen die oben beschreibene Methylierungsanalyse am intakten Kern-Oligosaccharid anstelle des Disaccharids durchgeführt, so fand man ein 1,3,5-Tri-O-azetyl-7-O-(N,N-dimethylcarbamoyl)-2,4,6,tri-O-methyl-Heptitol, 17 mit einer Retentionszeit von 24,5 min. Die Existenz eines 3-O-azetylierten 7-O-Carbamoyl-Heptitols in 17 kann durch die Substitution des GalN[Ala]-Restes in Position 3 von Hep II im intakten Kern-Oligosaccharid erklärt werden. Dieses Ergebnis steht in Übereinstimmung mit früher erhobenen Daten von Rowe und Meadow [Rowe, P.S.N. und Meadow, P.M., *Eur. J*. *Biochem., 132* (1983) 329-337] und E. Altman et al. (E. Altman et al., *Biochemistry* 1994, zur Publikation eingereicht; E. Altman et al., *Int. Carb. Conference,* Paris 1992, Abstract Book C161, S. 626; H. Masoud et al., 2nd Conference of the International Endotoxin Society (IES), Vienna 1992, 69, S. 55), welche den Substitutionsort der GalN-Ala und die Verknüpfung der Heptosen untereinander ebenfalls zu GalpN[Ala]-(1→3)-LαD-Hep*p*-(1→3)-L,D-Hep*p* bestimmt hatten.

### 4.4. Zuordnung der 7-O-Carbamoyl-Heptose zur L-glycero-D-manno- oder D-glycero-D-manno-Konfiguration

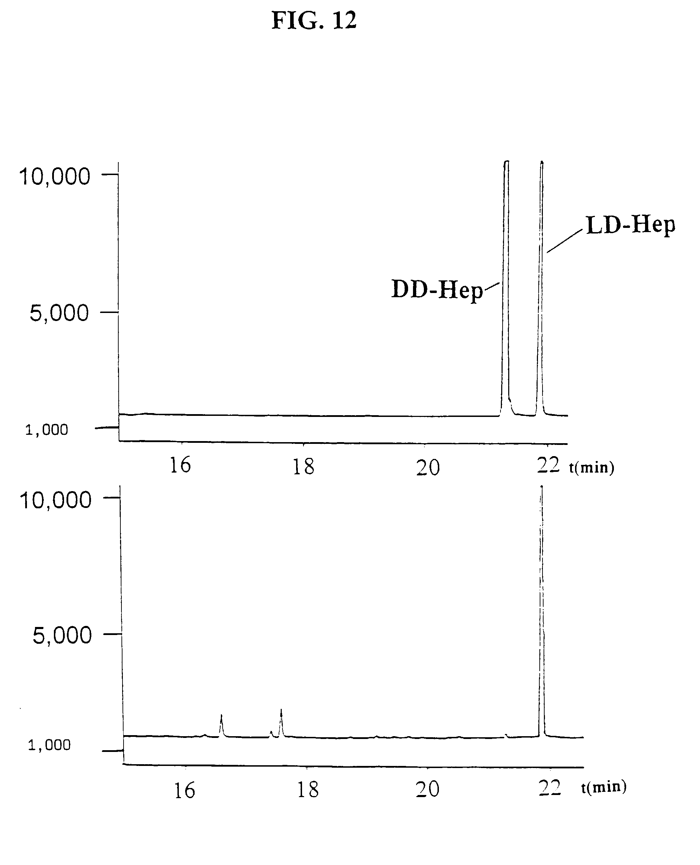

In der GLC-Analyse (SPB-5®) wurde zunächst ein Standard vermessen, der sowohl perazetyliertes D-glycero-D-manno-Heptitol (D,D-Hep-ol) als auch L-glycero-D-manno-Heptitol (L,D-Hep-ol) enthielt (t_{R} = 21,36 min, D,D-Hep: t_{R} = 21,89 min, L,D-Hep). Nachdem der 7-O-Carbamoyl-Rest der Hep II aus dem Kern-Oligosaccharid von *P. aeruginosa* PAC605 selektiv durch milde alkalische Hydrolyse entfernt worden war, wurden die so dargestellten Heptitol-Azetate aus Hep I und Hep II in der GC-Analyse untersucht. Es zeigte sich, daß nur L-glycero-D-manno-Heptitol-Azetat nachweisbar war, t_{R} = 21,89 min, was dem L,D-Hep-ol entspricht (Fig. 12). Aus diesem Experiment und der Tatsache, daß durch die Abspaltung des Carbamoylsubstituenten die Heptose intakt bleibt, konnte die L-glycero-D-manno-Heptose-Konfiguration für die 7-O-Carbamoyl-Heptopyranose (Hep II) wahrscheinlich gemacht werden (vgl. auch NMR-Analyse).

### 4.5 NMR-Analyse des Methyl-3-O-(7-O-Carbamoyl-L-glycero-α-D-manno-Heptopyranosyl)-L-glycero-α-D-manno-Heptopyranose, 9α

### 4.5.1. ¹H-NMR-Analyse

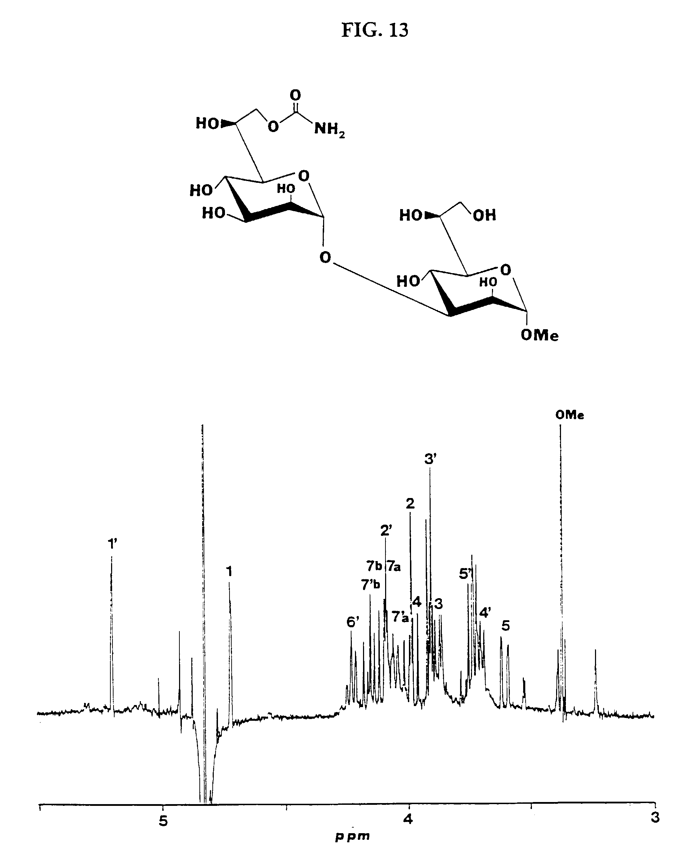

Das Disaccharid 3-O-(7-O-Carbamoyl-L-glycero-α-D-manno-Heptopyranosyl)-L-glycero-α-D-manno-Heptopyranose, 9α wurde nach milder Methanolyse aus dem dephosphorylierten Kern-Oligosaccharid isoliert und mittels HPLC gereinigt. Die Ergebnisse der Protonen-NMR-Analyse sind in Figur 13 und Tabelle 1 dargestellt. Signifikant ist die Tieffeldverschiebung der Signale von H-7'a und H-7'b um ca. 0,3 ppm (4,085 und 4,154 ppm) bei 7-O-Carbamoyl-Heptose, verglichen mit den analogen Signalen von H-7a und H-7b in der unsubstituierten Heptose (3,727 bzw. 3,751 ppm). Die Substitution in Position 3 Hep I manifestiert sich lediglich in einer Tieffeldverschiebung von ca. 0,12 ppm im Vergleich zum α-anomeren Methyl-L-glycero-D-manno-Heptopyranosid 2α. Alle anderen Signale sind in guter Übereinstimmung mit dem synthetischen Disaccharid Methyl-3-O-(L-glycero-α-D-manno-Heptopyranosyl)-L-glycero-α-D-manno-Heptopyranose (10α). Die gute Übereinstimmung der Signale ist ein weiteres Indiz für die in der GC-Analyse gefundene L-glycero-α-D-manno-Konfiguration.

### 4.5.2 ¹³C-NMR-Analyse von Disaccharid 9α

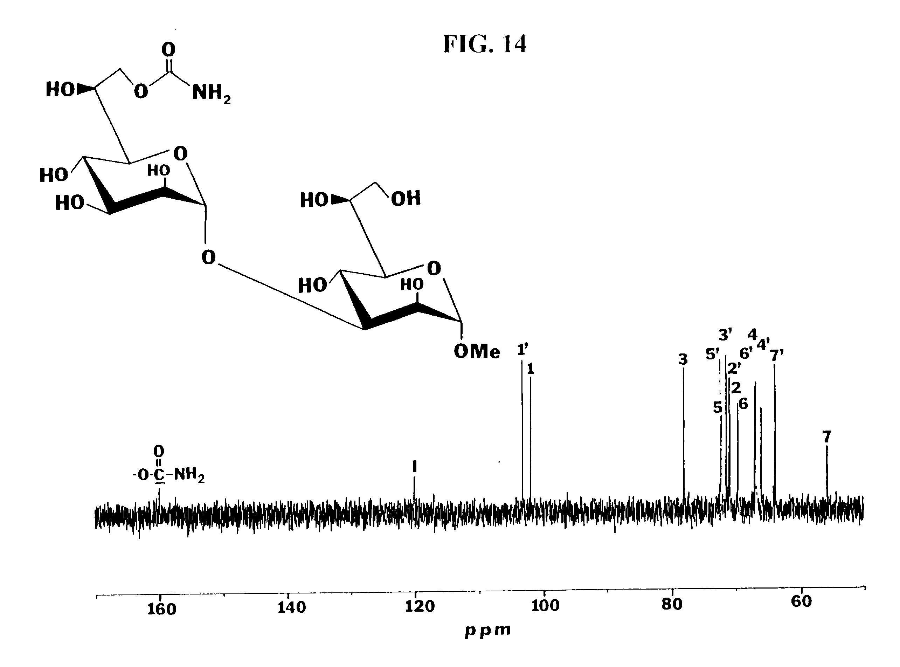

Die Ergebnisse der ¹³C-NMR-Analyse, wie sie aus den heteronuklear-korrelierten Spektren für das Disaccharid 3-O-(7-O-Carbamoyl-L-glycero-α-D-manno-Heptopyranosyl)-L-glycero-α-D-manno-Heptopyranose (9α) hervorgegangen sind, finden sich in Figur 14 und Tabelle 2. Durch den Vergleich mit dem synthetischen Disaccharid Methyl-3-O-(L-glycero-α-D-manno-Heptopyranosyl)-L-glycero-α-D-manno-Heptopyranose (10α) und dem synthetischen Monosaccharid Methyl-L-glycero-α-D-manno-Heptopyranosid (2α) konnten die einzelnen Strukturmerkmale der Heptose (7-O-Carbamoyl-Substitution, glycosidische Bindung und Konfiguration) abgeleitet werden. Besonders auffällig ist das Carbonyl CO-NH₂-Signal bei 159,86 ppm, welches in guter Übereinstimmung mit einem analogen Signal (159,6 ppm) einer 6-O-Carbamoyl-GlcN(Me,18:0)-R steht, die kürzlich aus *Azorhizobium caulinodans* (Mergaert. P. et al.. *Proc. Natl. Acad. Sci. USA, 90* (1993) 1551-1555) beschrieben wurde. Die Tieffeldverschiebung des Signals von C-7' bei 9α liegt nur ca 2,2 ppm ggenüber der unsubstituierten Verbindung 10α, was bei glycosidischen Substituenten an primären Hydroxylgruppen nicht ungewöhnlich ist. Signifikant dagegen ist die Tieffeldverschiebung von C-3 an Hep I (71,35 vs. 77,89 ppm), wodurch die Hep-(1→3)-Hep-Substitution im Disaccharid erneut bestätigt werden konnte. Alle anderen Signale liegen in guter Übereinstimmung mit dem Disaccharid 10α. Die gute Übereinstimmung der ¹³C-Signale mit den synthetischen Referenzverbindungen 2α und 10α ist ein weiteres Indiz für die gefundene L-glycero-α-D-manno-Konfiguration in beiden Heptosen.

### 4.6. Laser-Desorptions-Massenanalyse (LD-MS) des Methyl-3-O-(7-O-Carbamoyl-L-glycero-α-D-manno-Heptopyranosyl)-L-glycero-α-D-manno-Heptopyranose, 9α

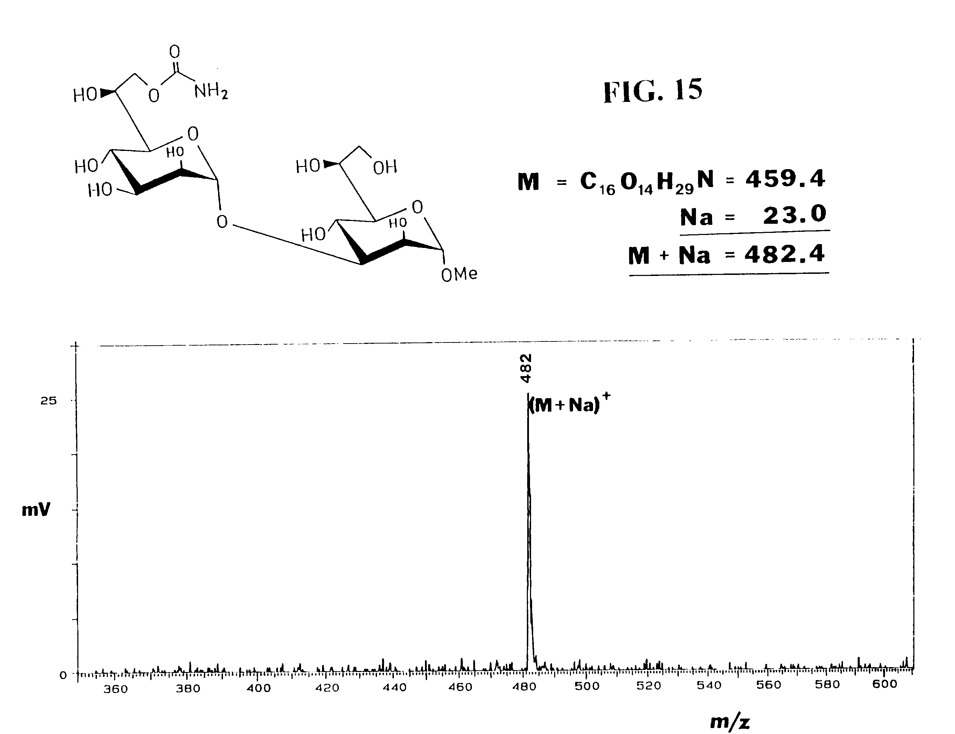

Das Ergebnis der Laser-Desorptions-Massenanalyse von Verbindung 9α ist in Fig. 15 dargestellt. Das Molekulargewicht der Methyl 3-O-(7-O-Carbamoyl-L-glycero-α-D-manno-Heptopyranosyl)-L-glycero-α-D-Heptopyranose (9α) wurde mit der Summenformel C₁₆O₁₄H₂₉N = 459,40 berechnet. Das LD-MS-Spektrum zeigte das gereinigte Disaccharid 9α als singulären pseudomolekularen Peak mit einer Molekülmasse von [M + Na]⁺ = 459 + 23 = 482 und steht damit in exzellenter Übereinstimmung mit der berechneten und gezeigten Struktur.

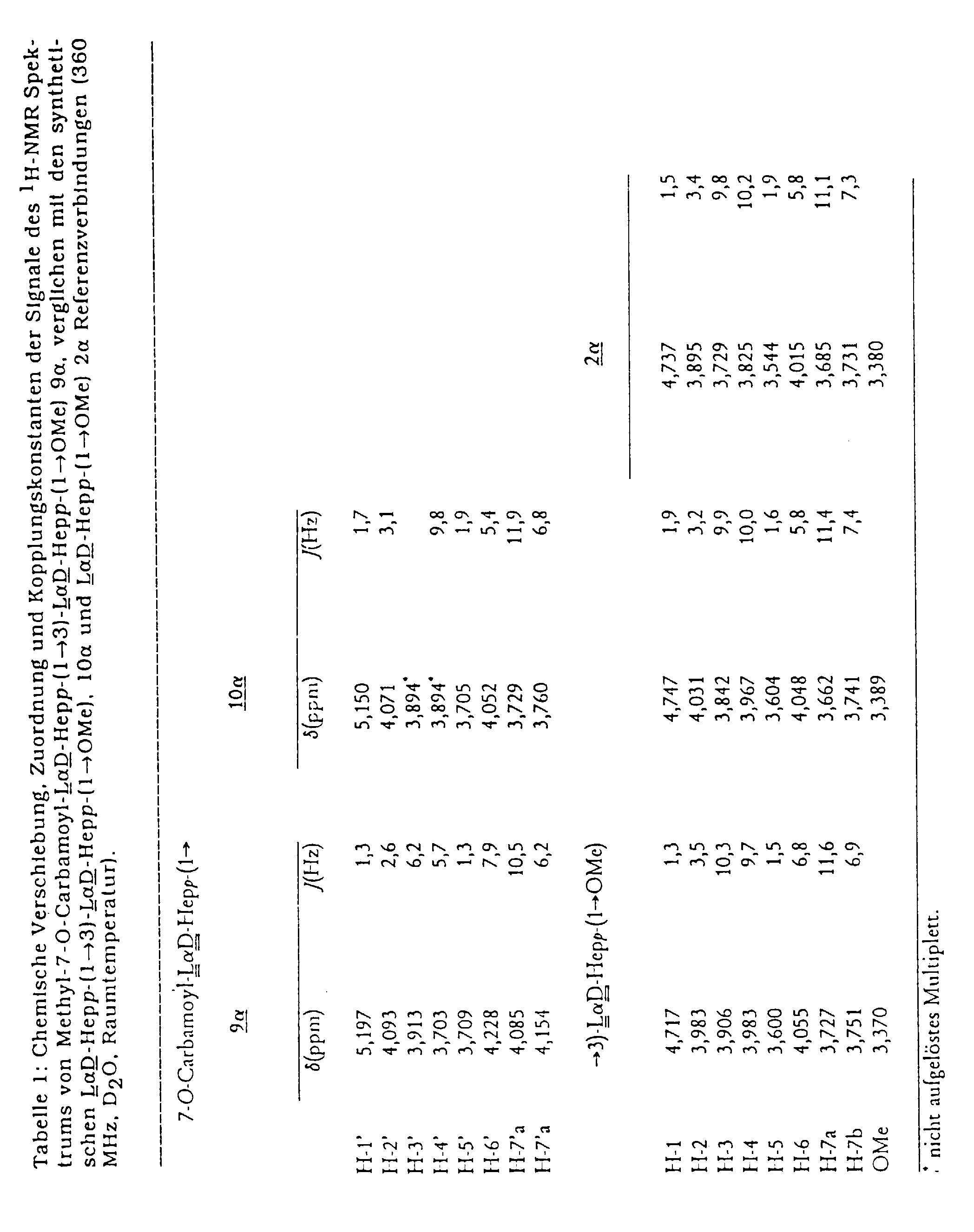

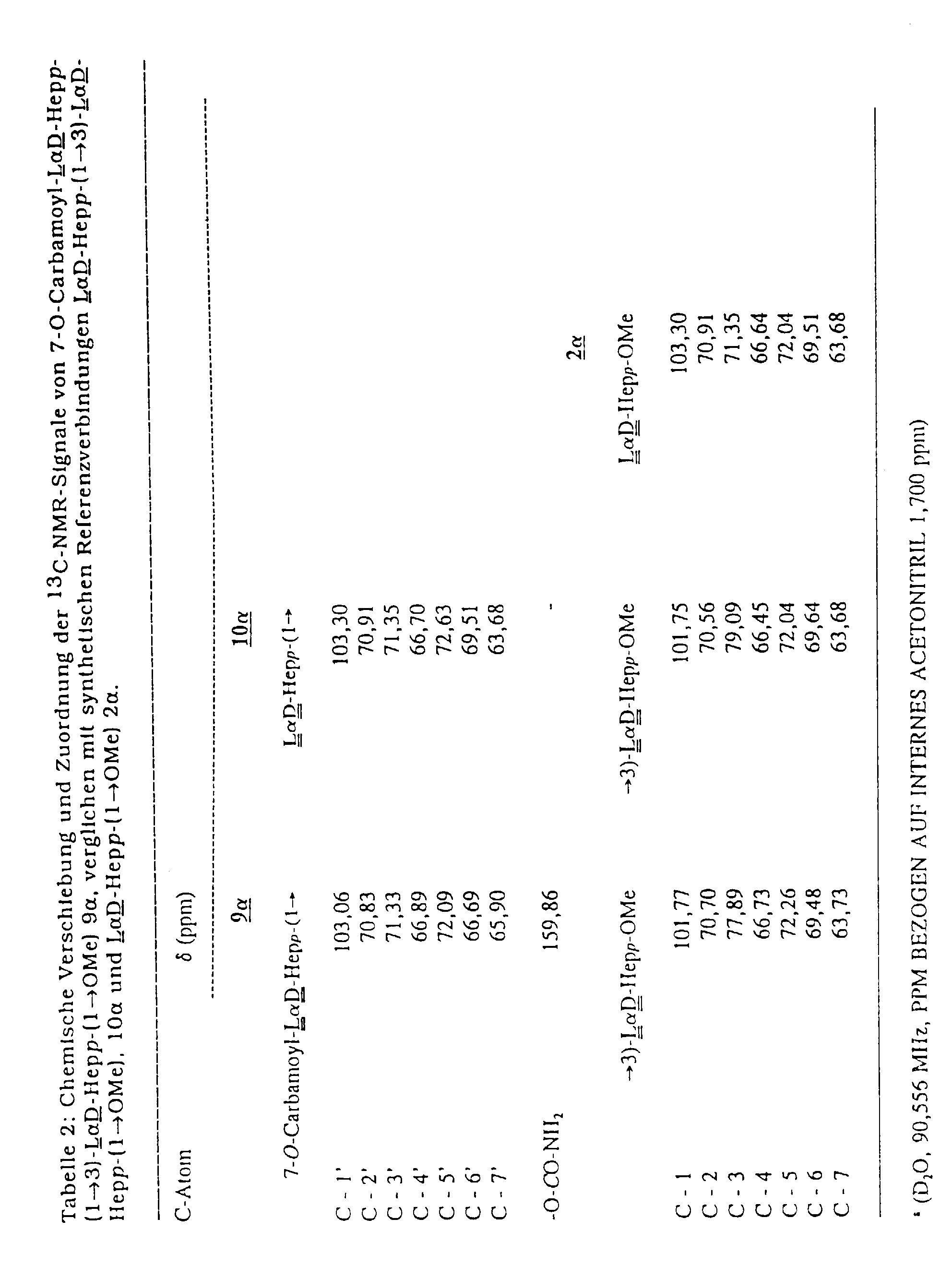

**Tabelle 3**

| **Identifizierung der 7-O-Carbamoyl-Heptose im Kern-Oligosaccharid von verschiedenen Gram-negativen Bakterien** **7-O-Carbamoyl-Heptopyranose** | |
|---|---|
| **1. Pseudomonadaceae** (alte Klassifikation) | |
| 1.1 Rauhform-Mutanten | |
| Pseudomons aeruginosa PAC605 | + |
| P. aeruginosa PAC 557 | + |
| P. aeruginosa PAC1R | + |
| P. aeruginosa R5 (Habs 6) | + |

| 1.2. Glattform-Bakterien | |
|---|---|
| P. aeruginosa Immunotyp Fischer 2 | + |
| P. aeruginosa Immunotyp Fischer 7 | + |
| P. aeruginosa 170519 | + |
| P. aeruginosa 170520 | + |
| P. aeruginosa FH-N-845 | + |
| | |
| P. fluorescens ATCC 49271 | + |
| | |
| Pseudomonas plantarii DSM 7128 | - |

| **2. Nicht-Pseudomonadaceae** | |
|---|---|
| Klebsiella pneumoniae, K 25 | - |
| Yersinis enterocolitica. Mutante 490 M | - |
| Campylobacter jejuni RN16 O:58, CCUG 10936 | - |
| Proteus mirabilis Mutante R₄₅, | - |
| Haemophilus influenzae, Wildtyp, Stamm Eagan | - |
| Vibrio parahaemolyticus, Serotyp O12 | - |
| Salmonella minnesota SF 1111 (S-Form) | - |
| Escherichia coli O111 (S-Form) | - |

## Patentansprüche

1. 7-O-Carbamoyl-Heptose-Derivate der allgemeinen Formel (I) 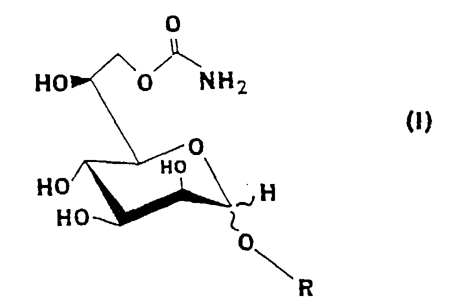 worin R den Substituenten R¹ oder eine Gruppe der allgemeinen Formel (II) bedeutet. 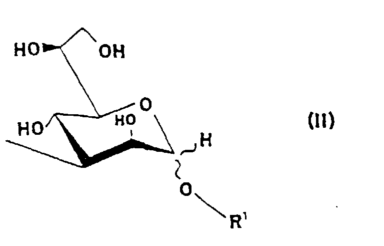 worin R¹ ein Wasserstoffatom, eine Methylgruppe oder ein für eine kovalente Kopplung geeigneter Linker-Substituent ist.

2. 7-O-Carbamoyl-Heptose-Derivate nach Anspruch 1, wobei der Linker-Substituent ein Cysteaminrest, eine Allylgruppe oder eine geradkettige oder verzweigtkettige C₁₋₁₈-Alkylgruppe, die eine endständige Hydroxyl-, Amino-, Carbonyl-, Carboxyl- oder Allylgruppe enthalten kann, ist.

3. 7-O-Carbamoyl-Heptose-Derivate nach Anspruch 1, **dadurch gekennzeichnet**, daß R¹ ein Wasserstoffatom oder eine Methylgruppe bedeutet.

4. Verfahren zur Herstellung der 7-O-Carbamoyl-Heptose-Derivate nach den Ansprüchen 1 bis 3, **dadurch gekennzeichnet**, daß man ein Lipopolysaccharid von Pseudomonaden der RNA-Gruppe 1 (u.a. P. aeruginosa, P. fluorescens, P. putida) mit Fluorwasserstoffsäure behandelt, das erhaltene Produkt bis zur Erreichung eines neutralen pH-Werts gegen Wasser dialysiert, das so dephosphorylierte Lipopolysaccharid methanolisiert, daran anschließend eine Permethylierung oder Perazetylierung durchführt, die erhaltenen 7-O-Carbamoyl-Heptose-Derivate durch Flüssigkeitschromatographie (unter Überprüfung der Reinheit durch Gas-Flüssigkeitschromatographie oder kombinierte Gas-Flüssigkeitschromatographie/Massenspektrometrie) auftrennt, und falls erwünscht, den Substituenten R¹ nach einem an sich bekannten Verfahren in die erhaltenen 7-O-Carbamoyl-Heptose-Derivate einführt.

5. Screening-Verfahren zur Bestimmung von 7-O-Carbamoyl-Heptose-Derivaten der Formel (I) wie in Anspruch 1 definiert in Gram-negativen Bakterien, **dadurch gekennzeichnet**, daß man intakte Bakterien ohne vorherige Abtrennung des Lipopolysaccharids mit Fluorwasserstoffsäure behandelt, danach zur Freisetzung der Heptose-Derivate hydrolysiert bzw. methanolisiert, daran anschließend eine Permethylierung durchführt und das permethylierte Produkt mittels Gas-Flüssigkeitschromatographie bzw. kombinierter Gas-Flüssigkeitschromatographie/Massenspektrometrie analysiert.

6. Verwendung der 7-O-Carbamoyl-Heptose-Derivate nach den Ansprüchen 1 bis 3 zur Herstellung von Reagenzien und Zusammensetzungen für die Diagnose und Therapie von Pseudomonasinfektionen bei Mensch und Tier.

## Claims

1. 7-O-Carbamoylheptose derivatives of the general formula (I) 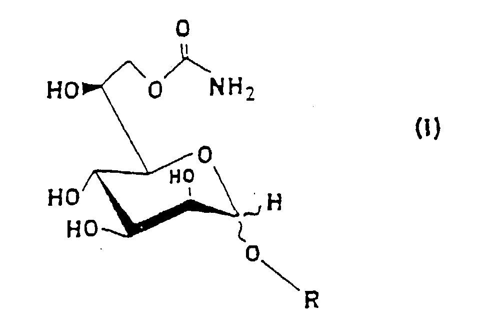 in which R is the substituent R¹ or a group of the general formula (II) 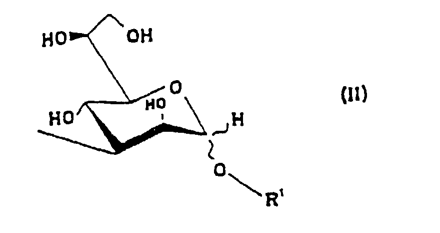 in which R¹ is a hydrogen atom, a methyl group or a linker substituent suitable for a covalent coupling.

2. 7-O-Carbamoylheptose derivatives according to claim 1, where the linker substituent is a cysteamine residue, an allyl group or a straight-chain or branched-chain C₁₋₁₈-alkyl group which may contain a terminal hydroxyl, amino, acyl, carboxyl or allyl group.

3. 7-O-Carbamoylheptose derivatives according to claim 1, characterized in that R¹ is a hydrogen atom or a methyl group.

4. A process for the production of the 7-O-carbamoylheptose derivatives according to claims 1 to 3, characterized in that a lipopolysaccharide of pseudomonads of RNA group 1 (inter alia P. aeruginosa, P. fluorescens, P. putida) is treated with hydrofluoric acid, the resulting product is dialyzed against water until a neutral pH is reached, the lipopolysaccharide which has been dephosphorylated in this way is methanolyzed, and subsequently a permethylation or peracetylation is carried out, the resulting 7-O-carbamoylheptose derivatives are fractionated by liquid chromatography (checking the purity by gas-liquid chromatography or combined gas-liquid chromatography/mass spectrometry) and, if required, the substituent R¹ is introduced into the resulting 7-O-carbamoylheptose derivatives by a process known per se.

5. A screening process for the determination of 7-O-carbamoylheptose derivatives of the formula (I) as defined in claim 1 in Gram-negative bacteria, characterized in that intact bacteria are treated, without previous removal of the lipopolysaccharide, with hydrofluoric acid, then hydrolyzed or methanolyzed to liberate the heptose derivatives, and subsequently a permethylation is carried out and the permethylated product is analyzed by gas-liquid chromatography or combined gas-liquid chromatography/mass spectrometry.

6. The use of the 7-O-carbamoylheptose derivatives according to claims 1 to 3 for the production of reagents and compositions for the diagnosis and therapy of pseudomonas infections in humans and animals.

## Revendications

1. Dérivés de 7-O-carbamyle-heptose de formule générale (I) 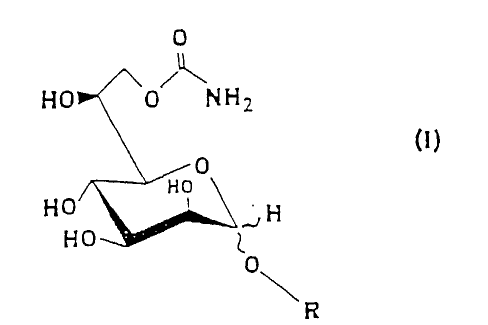 dans laquelle R correspond au substituant R¹ ou à un groupement de formule générale (II), 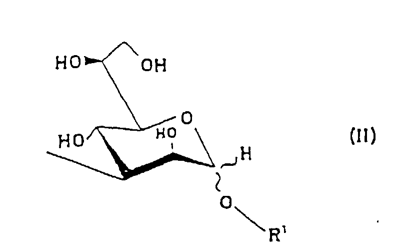 dans laquelle R¹ est un atome d'hydrogène, un groupement méthyle ou un substituant de liaison adapté à former une liaison covalente.

2. Dérivés de 7-O-carbamyle-heptose selon la revendication 1, où le subsituant de liaison est un résidu cysteamine, un groupement allyle ou un groupement alkyle C₁₋₁₈ à chaîne linéaire ou ramifiée qui peut contenir un groupement terminal hydroxyle, amine, carbonyle, carboxyle ou allyle.

3. Dérivés de 7-O-carbamyle-heptose selon la revendication 1, caractérisés en ce que R¹ correspond à un atome d'hydrogène ou un groupement méthyle.

4. Procédé de préparation de dérivés de 7-O-carbamyle-heptose selon les revendications 1 à 3, caractérisé en ce que l'on traite un lipopolysaccharide de pseudomonas du groupe RNA 1 (i.a. P.aeruginosa,P.fluorescens,P.putioloa) avec de l'acide fluorhydrique, on dialyse le produit obtenu avec de l'eau pour obtenir une valeur de pH neutre, on méthanolyse le lipopolysaccharide ainsi déphosphorylé, on réalise ensuite une perméthylation ou une peracétylation, les dérivés de 7-O-carbamyle-heptose obtenus étant séparés par chromatographie liquide (sous contrôle de la pureté par chromatographie gaz/liquide ou par combinaison chromatographie gaz/liquide et spectrométrie de masse), et si souhaité, le substituant R¹ est introduit dans le dérivé 7-O-carbamyle-heptose à l'aide d'un procédé connu en soi.

5. Procédé de dépistage pour détecter des dérivés de 7-O-carbamyle-heptose de formule (I) tels que définis dans la revendication 1 dans des bactéries Gram négatives, caractérisé en ce que l'on traite avec de l'acide fluorhydrique des bactéries intactes sans séparation préalable des lipopolysaccharides, puis on réalise une hydrolyse ou une méthanolyse pour libérer le dérivé heptose, on réalise ensuite sur celui-ci une perméthylation et on analyse le produit perméthylé par chromatographie gaz/liquide ou par une combinaison chromatographie gaz/liquide / spectrométrie de masse.

6. Utilisation de dérivés de 7-O-carbamyle-heptose selon l'une des revendications 1 à 3 pour la fabrication de réactifs et de compositions pour le diagnostic et la thérapie des infections de pseudomonas chez l'homme et l'animal.
